# EUROPEAN PATENT SPECIFICATION

(11) **EP 2 775 937 B1**
(45) Date of publication and mention of the grant of the patent: **20.07.2016**
(21) Application number: 12846903.8
(22) Date of filing: 12.11.2012
(51) Int. Cl.: A61B 17/04, A61B 17/86, A61B 17/11, A61F 2/08

(54) **TRANSOSSEOUS ATTACHMENT ANCHOR**
TRANSOSSEALES BEFESTIGUNGSANKER
ANCRAGE POUR FIXATION TRANSOSSEUSE

(30) Priority: 11.11.2011 US 201161558933 P; 16.11.2011 US 201161560694 P; 09.02.2012 US 201261597066 P; 09.02.2012 US 201261597138 P
(43) Date of publication of application: 17.09.2014
(73) Proprietor: Venture MD, Broomfield, Colorado 80038 (US)
(72) Inventor: CHAN, Kwan-Ho, Singapore, Singapore 126823 (SG); MURPHY, James, Newton Square, Pennsylvania 19073 (US); FALLIN, Thomas Wade, Logan, UT 84321 (US); WHITE, Patrick Michel, Avondale, Pennsylvania 19311 (US)
(74) Representative: Kransell & Wennborg KB
(86) International application number: PCT/US2012/064669
(87) International publication number: WO 2013/071234

(56) References cited:
- EP-A1- 2 070 481
- WO-A1-2010/132310
- US-A1- 2005 055 052
- US-A1- 2005 055 052
- US-A1- 2008 033 486
- US-A1- 2009 157 124
- US-A1- 2010 121 349
- US-B1- 6 517 542
- US-B2- 7 063 724
- US-B2- 7 175 632

## Description

### FIELD OF THE INVENTION

The invention relates to suture anchors for surgical transosseous attachments.

### BACKGROUND

A variety of surgical procedures require the attachment of something relative to a surgical site. For example, in surgery relating to the skeletal system, it is often advantageous to attach soft tissue, suture, implants, and/or other items in or adjacent to a joint. For example, ligaments, tendons, fascia, other capsular material, and/or muscle may be attached to an adjacent bone to effect a repair of a joint. Such joints may include any joint in a patient's body such as the joints of the hands and feet, ankle, wrist, knee, elbow, hip, shoulder, and spine. For example, it is often advantageous to pass a suture through a portion of a bone to form a transosseous attachment to the bone.

Document US 2005/055052 discloses a knotless suture anchor and a method for its insertion into tissue.

Document US 6 517 542 discloses a bone anchoring system comprising a bone screw and a mechanical insert in a central bore of the bone screw.

Document US 2010/121349 discloses devices and methods for locking and/or cutting tethers during a tissue modification procedure.

Document US 2009/157124 discloses an anchor assembly including an anchor defining a cavity and an opening to the cavity and an insertion member including a body having a proximal end portion and a flat distal end portion, and a head coupled to the proximal end portion of the body.

### SUMMARY

The present invention is defined in claim 1 and provides implants to attach items to a bone transosseously.

The invention may be used in a method of placing a suture transosseously through a bone includes forming a first bone tunnel in the bone; positioning a portion of a suture in the first bone tunnel; indexing a guide to the first bone tunnel; guiding a tunnel forming instrument with the guide to form a second bone tunnel transverse to and intersecting the first bone tunnel; and withdrawing the portion of the suture through the second bone tunnel.

The invention may be used in association with instruments for placing a suture transosseously through first and second transverse, intersecting bone tunnels include a guide and a tunnel forming instrument. The guide has a proximal end and a distal end. An indexing portion is formed nearer the distal end and is indexable to a first bone tunnel. The guide defines a guide path from nearer the proximal end to nearer the distal end in predetermined relationship to the indexing portion. The tunnel forming instrument is engageable with the guide in constrained guiding relationship along the guide path. The tunnel forming instrument has a distal end configured to penetrate bone and a suture retrieving portion having a feature able to receive a suture from the first tunnel for retrieval through the second tunnel.

In an aspect of the invention, a suture anchor includes a suture retaining feature or features able to retain first and second portions of a suture passed transosseously through a bone. For example, a suture passing through a bone may have first and second free portions and a single suture anchor according to the present invention may include a suture retaining feature able to secure both free portions of the suture to the bone. In another example, a single suture anchor may include multiple suture retaining features able to secure both free portions of the suture to a bone.

The portion of suture may include a single end, a pair of ends, a bight, or other portion of the suture.

The suture anchor includes a first body able to receive first and second portions of a suture in relative sliding relationship. A second body is receivable by the first body to lock the first and second portions relative to the first body. The second body may lock the first and second portions one at a time or simultaneously. The second body may lock the first and second portions at a single position on the first body or at separate discrete positions on the first body. For example, first and second portions of a suture may be placed through an opening in the first body and simultaneously locked by trapping the portions between the first and second body. In another example, first and second portions of a suture may be placed through separate openings in the first body and simultaneously locked. In another example, first and second portions of a suture may be placed through separate openings in the first body and sequentially locked.

### BRIEF DESCRIPTION OF THE DRAWINGS

Various examples of the present invention will be discussed with reference to the appended drawings. These drawings depict only illustrative examples of the invention and are not to be considered limiting of its scope.
FIG. 1 is a side elevation view of a set of instruments ;
FIG. 2 is a detail view of a portion of one of the instruments of FIG. 1;
FIG. 3 is a detail view of a portion of one of the instruments of FIG. 1;
FIGS. 4-11 are partial side sectional views of the set of instruments of FIG. 1 in use to pass a suture through a bone;
FIGS. 12 and 13 are partial side sectional views of a set of instruments similar to that of FIG. 1 illustrating an alternative arrangement for one of the instruments;
FIG. 14 is a partial side sectional view of a set of instruments similar to that of FIG. 1 illustrating an alternative arrangement for one of the instruments;
FIG. 15 is a perspective view illustrating an alternative arrangement for one of the instruments of FIG. 1;
FIG. 16 is a partial side sectional view illustrating an alternative arrangement for one of the instruments of FIG. 1;
FIG 17 is a perspective view of an instrument;
FIG. 18 is a side elevation view of the instrument of FIG. 17;
FIG. 19 is a side elevation view of the instrument of FIG. 17, opposite the view of FIG. 17;
FIGS. 20-25 are side section views of the instrument of FIG. 17 in use to pass a suture through a bone;
FIG. 26 is a side section view illustrating a method of fixing a suture to a bone using the instruments of FIG. 1;
FIG. 27 is a side section view, rotated 90 degrees from that of FIG. 26;
FIG. 28 is a top plan view of the method of FIG. 26;
FIG. 29 is a side section view illustrating a method of reinforcing bone tunnels formed with instruments similar to those of FIG. 1;
FIGS. 30 and 31 are partial side section views illustrating modifications to instruments of FIG. 1 to allow them to simultaneously form bone tunnels and reinforce the bone tunnels;
FIG. 32 is a side elevation view of an alternative suture useable with the instruments of FIG. 1;
FIG. 33 is a side section view illustrating the use of the suture of FIG. 32;
FIG. 34 is a partial side sectional view of an illustrative implant and method ;
FIGS. 35 and 36 are partial side sectional views of an illustrative implant and method ;
FIGS. 37 and 38 are partial side sectional views of an illustrative implant and method ;
FIG. 39 is a partial side sectional view of an illustrative implant and method ;
FIG. 40 is a partial side sectional view of an illustrative implant and method ;
FIG. 41 is a partial side sectional view of the implant of FIG. 40 in use in an alternative method;
FIG. 42 is a partial side sectional view of an illustrative implant and method ;
FIG. 43 is a perspective view of an illustrative implant according to the present invention;
FIG. 44 is a perspective view of the implant of FIG. 43;
FIG. 45 is a side elevation view of the implant of FIG. 43;
FIGS. 46-48 are sectional views taken along line 46-46 of FIG. 45 showing an aspect of the operation of the implant of FIG. 43;
FIGS. 49-53 are partial side sectional views showing a method of using the implant of FIG. 43; and
FIG. 54 is a partial side sectional view of the implant of FIG. 43 in use in an alternative method.

### DESCRIPTION OF THE ILLUSTRATIVE EXAMPLES

Minimally invasive surgery is surgery used to gain access to deeper parts of the human body through small incisions. Such surgery may range from mini-open surgery to arthroscopic surgery. Mini-open surgery is generally understood to mean surgery performed through small incision(s) under direct vision as opposed to arthroscopic (or endoscopic) surgery where surgery is performed through one or more stab incisions in which the arthroscope (or endoscope) is used for visualization. In arthroscopic surgeries, the size of the stab incisions generally ranges from 1 mm to 10 mm. The illustrative examples depict arthroscopic surgical techniques but it is to be understood that the techniques could be performed in any minimally invasive or open technique. The following illustrative examples depict instruments and techniques to pass a suture through a portion of the head of the humeral bone at the shoulder of a human patient to repair damaged soft tissue associated with the shoulder joint. Instruments and techniques described herein may be used to pass a suture through any bone, at surgical sites anywhere in a patient's body, and for any purpose. The terms "suture" and "suture strand" are used herein to mean any strand or flexible member, natural or synthetic, able to be passed through a bone tunnel and useful in a surgical procedure. The term "transverse" is used herein to mean to cross at an angle; i.e. not parallel. The term includes, but is not limited to right angles. The term "bight" is used herein to mean a bend or loop formed in the intermediate portion of a suture.

A human left shoulder joint is used to provide context for illustrative examples of a surgical technique. The subacromial space, between the humeral head and the undersurface of the acromion, is a potential space for surgical repair. This space is partially occupied by the subacromial bursa. Soft tissue layers overlie the shoulder joint. These layers define a soft tissue zone including the skin, subcutaneous tissue, muscles and bursal tissue. Instruments are inserted through the soft tissue zone via stab incisions and access canulae can be inserted through these stab incisions to facilitate the insertion and withdrawal of surgical instruments. The thickness of this soft tissue zone varies by patient and by location from a few millimeters to several centimeters.

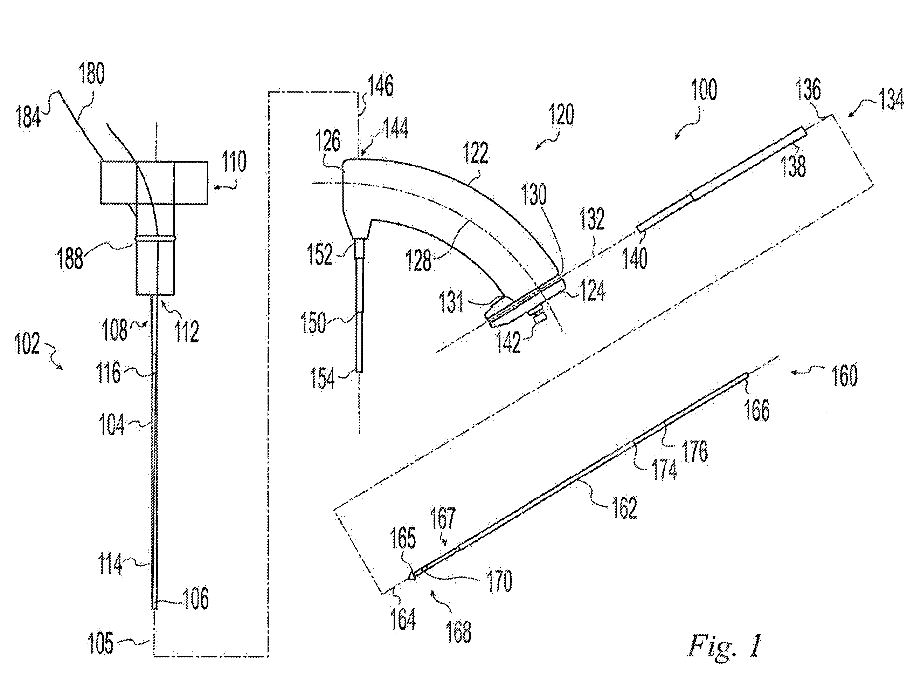

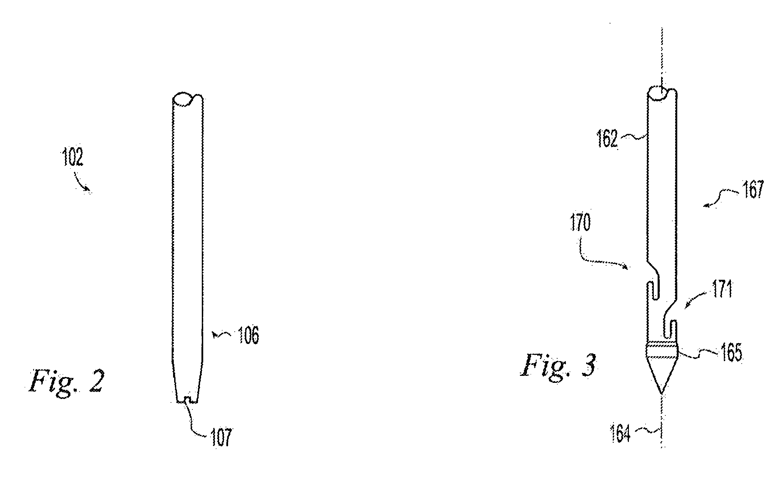

Referring to FIG. 1, a set of instruments 100 includes a punch 102, a tunnel guide 120, and a retriever 160. The punch 102 includes an elongated shaft 104 extending from a first, distal end 106 to a second, proximal end 108 along an axis 105. A punch handle 110 includes an opening 112 for receiving the proximal end 108. The punch handle 110 is able to lock onto the punch 102, e.g. with a rotating collet arrangement, to facilitate inserting the distal end 106 of the punch into tissue and removing it from tissue. The distal end 106 includes a notch 107 to aid in retaining a suture on the end as the suture is impacted into a bone as shown in FIG. 2.

The tunnel guide 120 includes an arm 122 extending from a first end 124 to a second end 126 along an axis 128. In the illustrative example of FIG. 1, the axis is arcuate. The first end 124 defines a passage 130 having an axis 132 transverse to the arm axis 128 and open along a slot 131 on one side. A retriever sleeve 134 has a longitudinal axis 136 extending between a first, proximal end 138 and a second, distal end 140. The retriever sleeve 134 is received in the passage 130 in axial translating relationship and is infinitely adjustable along its axis relative to the passage 130. Preferably the sleeve 134 fits closely within the passage 130 and is larger than the slot 131 so that the sleeve cannot pass sideways out of the passage 130. A lock 142 is operable to lock the retriever sleeve 134 position relative to the passage 130. In the illustrative example, the lock 142 is a screw threaded into the first end 124 adjacent and transverse to the passage axis 132 and in communication with the passage 130 such that it is responsive to inward threading to advance toward and press against the retriever sleeve 134 and lock it in place. The second end 126 defines a passage 144 through the arm 122 having a longitudinal axis 146 transverse to the arm axis 128 and generally coplanar with the passage axis 132. The axes 146 and 132 converge on the concave side of the arcuate arm axis 128.

The exemplary guide 120 has a non-adjustable arm 122 maintaining the axes 132 and 146 in fixed angular relationship. The arm 122 may also include an adjustment mechanism allowing the angle between the axes 132 and 146 to be adjusted by the user to accommodate variations in patient anatomy and placement technique.

An indexing feature is provided to register the guide 120 to a first bone tunnel in preparation for forming a second bone tunnel in known relationship to the first bone tunnel. In the illustrative example of FIGS. 1-11, the indexing feature is in the form of a punch Sleeve 150. The punch sleeve 150 has a proximal end 152, a distal end 154, and an axial passage extending between the proximal and distal ends. The punch sleeve 150 is mounted coaxially in the passage 144 and extends distally from the arm 122 such that the punch sleeve 150 and passage 130, and consequently the sleeve 134 when it is received in the passage 130, converge distally.

The retriever 160, includes an elongated shaft 162 extending along an axis 164 from a first, proximal end 166 to a second, distal end 168. The distal end 168 has a distal, leading segment 165 and a proximal, narrowed segment 167 in which the diameter of the narrowed segment 167 is generally less than the maximum diameter of the leading segment 165. When the retriever 160 is inserted into bone, a bony tunnel is created in accordance to the maximum cross-sectional profile of the leading segment 165. The resistance to withdrawal of the impacted retriever 160 is reduced as there is reduced or no friction between the wall of the bone tunnel and the narrowed segment 167. A notch 170 is formed in the side of the shaft 162 near the distal end 168. The retriever is sized to fit within the retriever sleeve 134 and to pass sideways out of the passage 130 through the slot 131 when the retriever sleeve is not positioned in the passage 130.

FIG. 3 illustrates one example of the notch 170. In this illustrative example, the notch 170 is formed into the side of the shaft transverse to the axis 164 and then extends distally parallel to the axis 164. Additional notches, such as illustrative second notch 171, may be formed on the shaft to aid in suture retrieval. In the illustrative example of FIG. 3, a second notch 171 is formed 180 degrees and distally from the first notch 170. The one or more notches may be located within the narrowed segment 167 to reduce the likelihood of the proximal edges of the notches 170, 171 snagging against the wall of the bone tunnel.

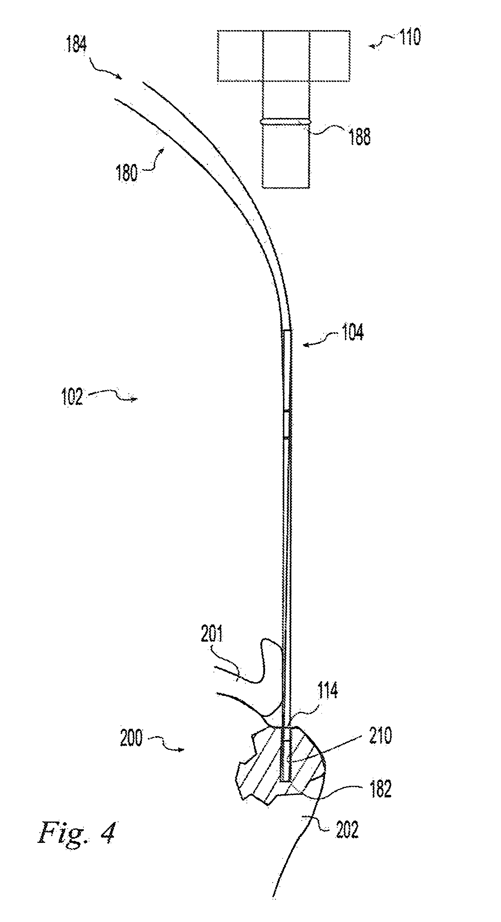

FIGS. 4-10 illustrate an exemplary technique using the instruments of FIG. 1. The punch is setup as shown in FIG. 1. The punch handle 110 is attached to the punch shaft 104. A suture 180 is mounted to the punch 102 by passing a portion 182 of the suture over the distal end 106 of the punch in the notch 107 and positioning the ends 184 of the suture toward the proximal end 108 of the punch. The ends 184 may be retained and/or placed in tension by engaging them with a retention mechanism such as a slot, boss, O-ring, and/or other mechanism into which or around which they may be positioned to maintain the suture 180 on the punch 102. For example, in the example of FIGS. 1-11, an O-ring 188 is mounted on the handle 110 and the suture 180 is run under the O-ring 188 to retain the suture 180.

The punch 102 is inserted into bone tissue. This may be accomplished by optionally predrilling the bone and then inserting the punch, or, as illustrated in the exemplary technique of FIGS. 2-8, the punch 102 may be inserted directly into the bone such as for example by impacting the handle 110 with a mallet to drive the distal end 106 into the bone.

In the exemplary technique of FIGS. 4-11, a shoulder 200 repair surgical procedure is illustrated such as for, for example, attaching soft tissues of the rotator cuff 201 to the proximal humerus 202. The punch 102 and suture 180 are introduced into the surgical site and the distal end 106 of the punch 102 is placed against the proximal humerus 202 at a desire suture exit location. The punch 102 and suture 180 may be inserted percutaneously through a small stab incision.

As shown in FIG. 4, the punch 102 and suture 180 are impacted into the bone tissue by impacting the punch handle 110 to drive the distal end 106 to a predetermined depth into the bone and to form a first bone tunnel 210. In the illustrative technique, the punch and suture are driven approximately 18mm into the bone. An index mark 114 may be provided a predetermined distance from the distal end 106 as a reference. The handle 110 is unlocked and removed from the punch shaft 104.

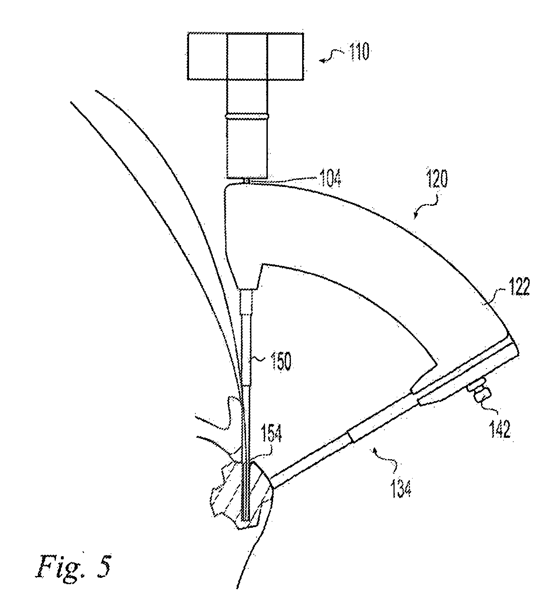

Referring to FIG. 5, the tunnel guide 120 is engaged with the punch shaft 104 by sliding the distal end 154 of the punch sleeve 150 over the proximal end 108 of the shaft 104 and advancing the tunnel guide 120 toward the bone until distal end 154 of the punch sleeve 150 is brought to rest against the bone. The handle 110 is remounted to the punch shaft 104. The retriever sleeve 134 is engaged with the passage 130 in the arm 122 and advanced percutaneously into the surgical site until it rests against the bone. The lock 142 is actuated to lock the sleeve 134 in place.

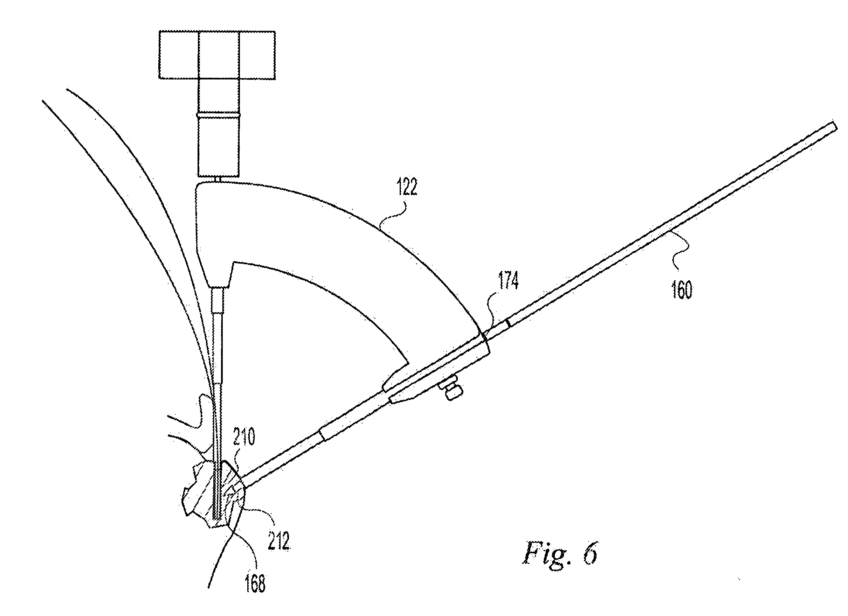

Referring to FIG. 6, the retriever 160 is advanced toward the punch 102. This may be accomplished by optionally predrilling the bone through the retriever sleeve and then inserting the retriever 160, or the retriever 160 may be inserted directly into the bone. In the illustrative example of FIG. 6, the retriever 160 is introduced to the surgical site through the retriever sleeve 134 until the distal end 168 is adjacent the bone. It is then impacted into the bone forming a second bone tunnel 212. It is impacted to a first predetermined depth in which the distal end 168 is spaced from the punch shaft 104. An index mark 174 on the retriever shaft 162 may be provided a predetermined distance from the distal end 168 as a reference. For example, the index mark 174 may be referenced to the arm 122 to indicate that the predetermined depth has been reached.

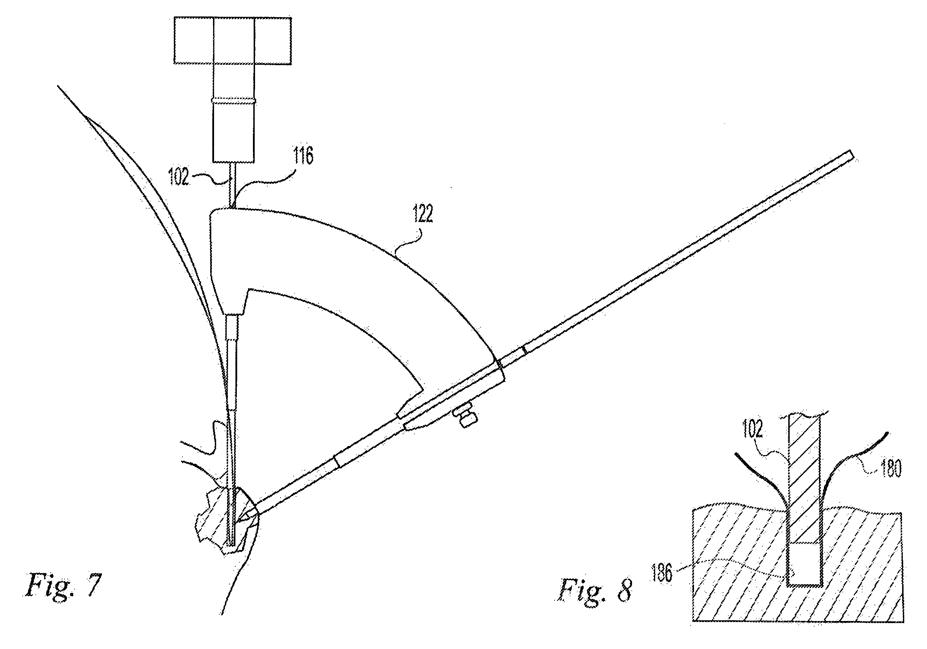

Referring to FIGS. 7 and 8, the punch 102 is withdrawn a predetermined distance to provide clearance for the retriever. An index mark 116 may be provided a predetermined distance from the distal end 106 as a reference. For example, the index mark 116 on the punch shaft 104 may be referenced to the arm 122 to indicate that withdrawal the predetermined distance has been accomplished. FIG. 8 is a cross sectional view looking along the axis of the retriever toward the punch 102. The suture 180 is pressed into the bone and adheres to the bone when the punch 102 is driven such that when the punch 102 is withdrawn, the suture 180 remains in the depth of the punched hole forming a loop 186.

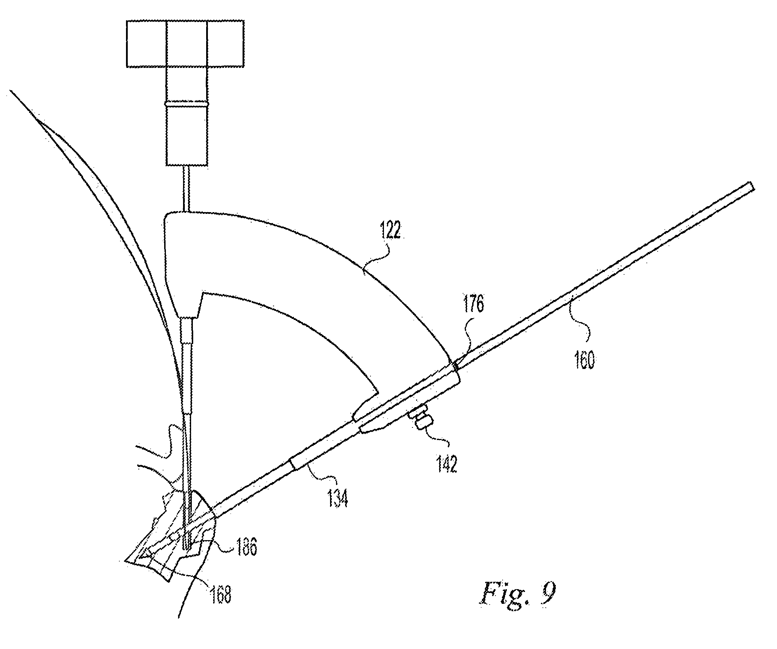

Referring to FIG. 9, the retriever 160 is then advanced further into the bone to a second predetermined depth in which the distal end 168 intersects the loop 186 of suture. An index mark 176 may be provided to indicate the second predetermined depth. The lock 142 is now disengaged to release the sleeve 134 and the sleeve 134 is withdrawn proximally from the passage 130 and removed. The arm 122 may now be rotated away from the retriever so that the retriever exits the passage 130 via the slot 131 thus freeing the arm 122, punch sleeve 150, and punch102 to be withdrawn axially away from the bone and removed from the surgical site.

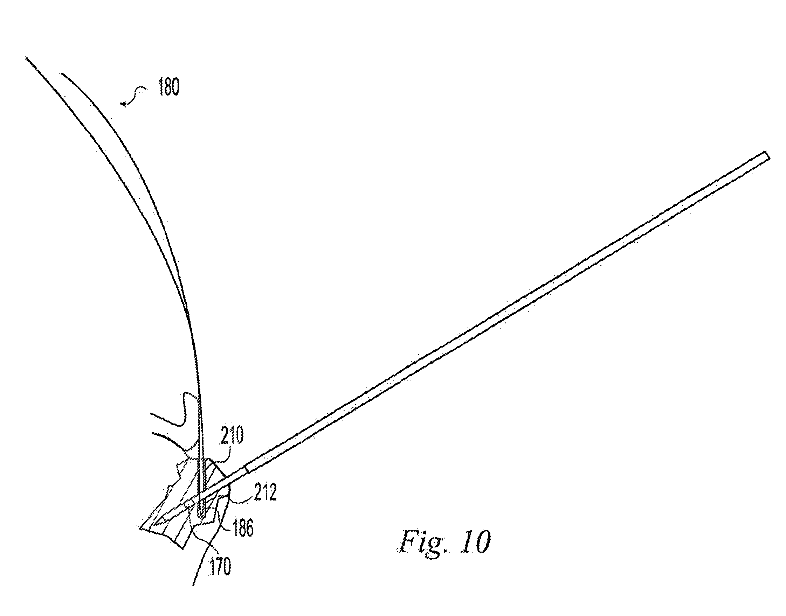

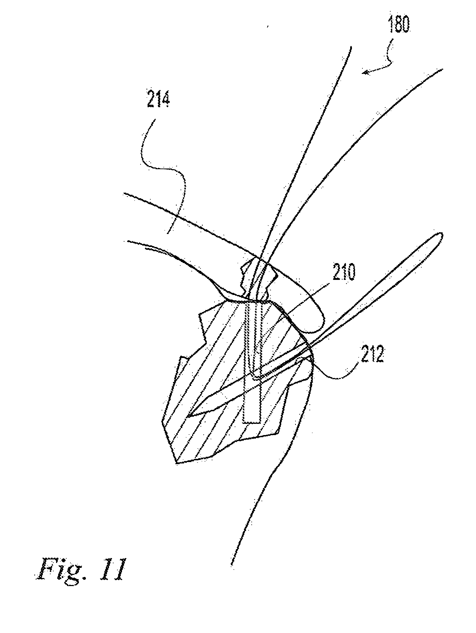

Referring to FIGS. 10 and 11, the suture 180 is tensioned to pull the loop 186 against the side of the retriever. The retriever is withdrawn so that the notch 170 engages the suture loop 186 and pulls the suture loop 186 through the second bone tunnel 212 such that the suture passes through the bone and extends outwardly from both the first and second bone tunnels 210, 212. The suture 180 may now be passed through soft tissue 214 adjacent the bone and used to connect the soft tissue to the bone such as by tying the suture, passing it over a button, and/or some other suture fastening technique.

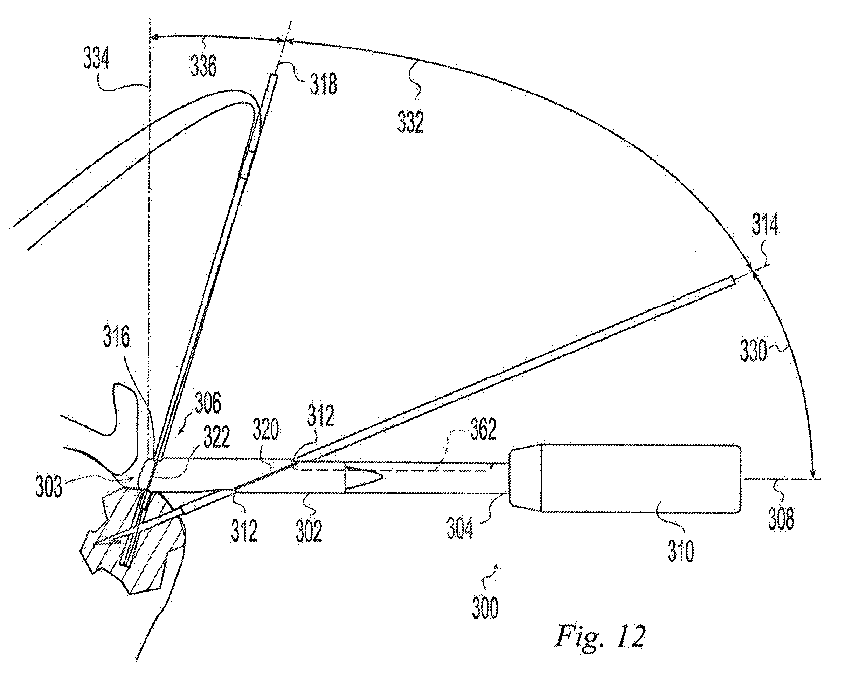

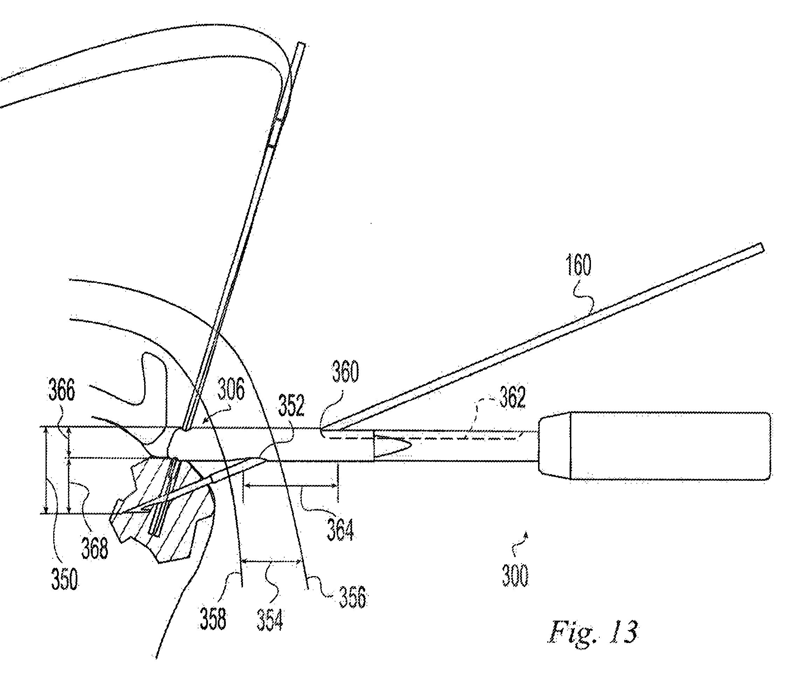

FIGS. 12 and 13 depict an illustrative set of instruments similar to the example of FIG. 1 that is modified to have a reduced size. A tunnel guide 300 includes a shaft 302 extending from a first, proximal end 304 to a second, distal end 306 along an axis 308. The distal end 306 defines a head member 307. A handle 310 is mounted to the proximal end 304. A retriever guide passage 312 is formed transversely through the shaft 302 along an axis 314. An indexing feature in the form of a punch guide passage 316 is formed transversely through the head member 307 along an axis 318 distal to the retriever guide passage 312. Index marks 320 and 322 are provided on the exterior of the shaft aligned with and parallel to the passage axes 314, 318 to aid in visualizing the passage paths.

The retriever guide passage 312 forms an angle 330 with the shaft axis 308. The retriever guide passage angle 330 is between 0 and 90 degrees, preferably between 10 and 30 degrees. In the illustrative example of FIGS. 12 and 13, the retriever guide passage angle 330 is approximately 20 degrees. Likewise, the punch guide passage 316 forms an angle 332 with the retriever guide passage 312. The angle between the passage axes 318, 314 is preferably between 0 and 90 degrees, more preferably between 30 and 70 degrees. In the illustrative example of FIGS. 12 and 13, the punch guide passage 316 is angled approximately 50 degrees relative to the retriever guide passage 312. Referenced in a different way, in the illustrative example of FIGS. 12 and 13, the punch guide passage is angled proximally at an angle 336 of approximately 20 degrees from a reference line 334 normal to the shaft axis 308.

Optionally, a groove 362 is formed in the shaft 302 and extends lengthwise along a portion of the shaft 302 until it intersects the external exit opening 360. The groove 362 facilitates locating the retriever guide passage even if the retriever guide passage is covered by soft tissue such as the margins of the percutaneous portal to the surgical site. For example, the retriever 160 may be positioned by sliding the distal end of the retriever distally along the groove, and under the soft tissue as necessary, until the retriever guide passage is engaged at the external exit opening 360. The retriever 160 may then be advanced towards the humeral head. The groove 362 allows the retriever 160 to be placed tactilely without direct visualization where anatomy or technique requires.

Referring to FIG. 13, optionally, but advantageously sharp portions of the lead profile are minimized to reduce the risk of snagging soft tissue during insertion. A soft tissue zone 354 is defined as the space between the skin 356 and soft tissues 358 near the bone; e.g. the humeral head. The axial spacing of the internal and external exit openings 352, 360 of the retriever guide passage 312 defines a protected guide path 364 over which the surrounding soft tissue is protected from the retriever 160. Optionally, but advantageously, the internal exit opening 352 on the tunnel guide is placed in close proximity to the surgical site to maximize the protected guide path 364 and avoid interference with the soft tissue zone 354 near the surgical site. Optionally, but advantageously, the external exit opening 360 on the tunnel guide is placed as proximal (nearer the proximal handle portion) as possible to maximize the protected guide path 364 and better accommodate large or obese patients having a wide soft tissue zone 354.

One way to move the internal exit opening 352 close to the surgical site is by reducing the lead profile height 350 which is defined as the vertical distance from the superior surface of the distal end 306 of the tunnel guide 300 to the inferior aspect of the distal end of the retriever 160 when it is inserted to its full operating depth in the bone. An indexing height 366 is defined as the vertical distance from the superior surface of the distal end 306 of the tunnel guide 300 to the inferior surface of the distal end 306 of the tunnel guide 300. A vertical bone depth 368 is defined as the vertical distance from the inferior surface of the distal end 306 of the tunnel guide 300 to the inferior aspect of the distal end of the retriever160 when it is inserted to its full operating depth in the bone. In the illustrative example of FIG. 13, the indexing height 366 and vertical bone depth 368 stack up to make up the lead profile height 350. Although the distance from the internal exit opening to the surgical site is preferably minimized, there is a limit because a minimum angle between the first and the second bone tunnels needs to be maintained to ensure a sufficiently long suture bridge in the bone to provide adequate suture pull out strength. There is also a desired minimum vertical bone depth to create a first bone tunnel of a predetermined depth. However, the indexing height 366 can be reduced to decrease the lead profile height.

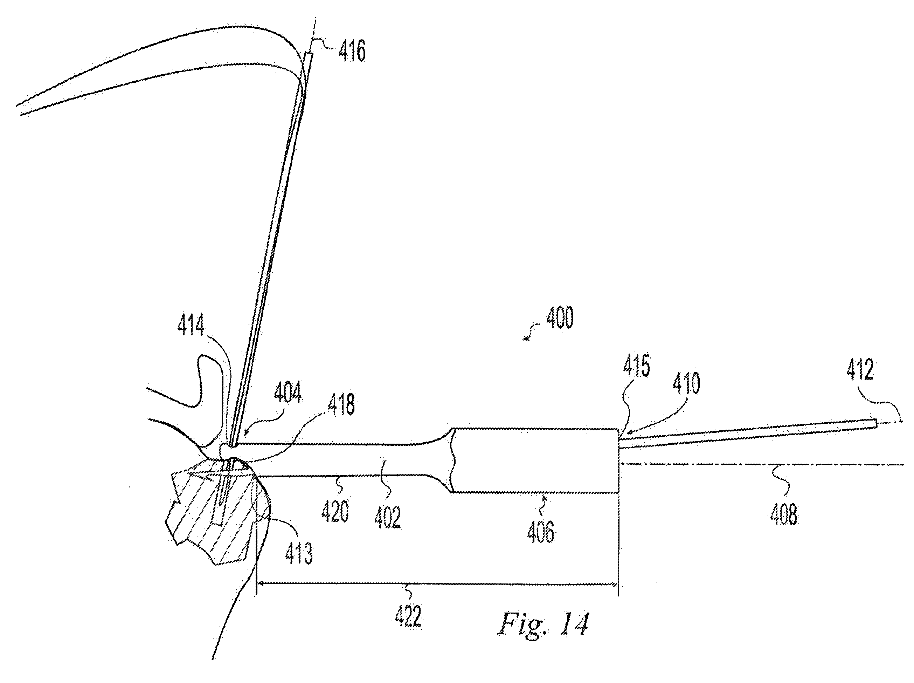

FIG. 14 illustrates another example of a tunnel guide 400 similar to that of FIGS. 12 and 13 but with a further reduced indexing height. The tunnel guide 400 includes a shaft 402 extending from a distal head member 404 to a proximal handle member 406 along an axis 408. A retriever guide passage 410 is formed transversely through the shaft member and the handle member along a retriever guide passage axis 412 and defines internal and external exit openings 413, 415. The retriever guide passage axis 412 forms an angle with the shaft axis 408.

An indexing feature 414 is formed transversely through the head member 404 along an indexing feature axis 416. The indexing feature axis 416 also forms an angle with the retriever guide passage axis 412. In the illustrative example of FIG. 14, the indexing feature is a punch guide passage. The passage may be a simple cylinder or have one or more additional slots formed on the sidewall of the passage to provide clearance for sutures alongside the punch. The indexing feature may take other suitable forms such as an open notch or a probe tip. In the illustrative example of FIG. 14, the inferior surface 418 of the head member 404 is superior to the inferior surface 420 of the shaft member whereas in the illustrative example of FIG. 13, the inferior surface of the head member is level with the inferior surface of the shaft member. Preferably, the inferior surface 418 of the head member as illustrated in Figure 14 has a curvature which approximately fits the contour of the bone at the surgical site. For example, for use in rotator cuff repair surgery, the curvature would fit the greater tuberosity of the humeral head such that the internal exit opening 413 of the tunnel guide is in close proximity to the surgical site. When the head member of the tunnel guide is thinner than the shaft member, as shown in FIG. 14, the internal exit opening 413 can be kept close to the surgical site while decreasing the angle between the tunnel guide axis 408 and the retriever axis 412. As a result, the external exit opening 415 may be located on the proximal end of the handle member 406 of the tunnel guide and the protected guide path 422 is lengthened as compared to the example of FIG. 10 and can accommodate an even wider soft tissue zone.

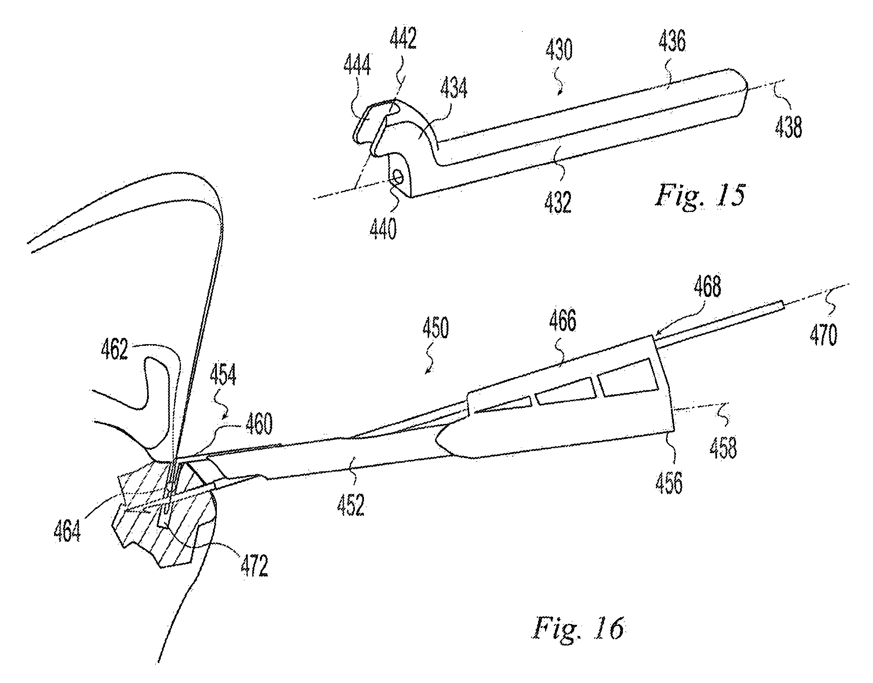

FIG. 15 illustrates another example of a tunnel guide 430 which lengthens the protected guide path to the maximum, without over-thinning of the head member. In the illustrative example in FIG. 12, the tunnel guide 430 includes a shaft member 432 extending from a distal head member 434 to a proximal handle member 436 along a tunnel guide axis 438. The distal head member 434 of the tunnel guide is curved. The undersurface of the distal head member is contoured to approximately fit the greater tuberosity of the humeral head.

A retriever guide passage 440 is formed through the shaft member 432 and the handle member 436 coaxial with the tunnel guide axis 438. When the head member of the guide is positioned on the greater tuberosity, the tunnel guide axis 438 is approximately level with the surgical site.

An indexing feature is formed transversely to the head member along an indexing feature axis 442. The indexing feature axis 442 forms an angle with the tunnel guide axis 438. In the illustrative example of FIG. 15, the indexing feature is a U-shaped slot 444 engageable with a tunnel forming instrument such as the punch of FIG. 1. Because the U-shaped slot 444 is open on one side, the first bone tunnel can be formed before the guide 430 is introduced to the surgical site. The guide can then be engaged with the tunnel forming instrument by introducing it, e.g., through a second soft tissue portal and sliding it sideways into engagement with the tunnel forming instrument. The procedure may then be carried out as described relative to the other examples. Alternatively the indexing feature can also be a punch guide passage that is engaged axially over the end of the first tunnel forming instrument to index the guide as in the previous examples or the indexing feature may be a probe engageable with a first bone tunnel upon removal of the first bone tunnel forming instrument to index the guide to position it for forming the second bone tunnel and retrieving a suture.

FIG. 16 illustrates another illustrative example of a tunnel guide. The tunnel guide 450 includes a shaft member 452 extending between a distal head member 454 and a proximal end 456 along a shaft axis 458. The distal head member 454 has an indexing feature in the form of a blunt-ended probe engageable with a first bone tunnel. The blunt-ended probe comprises a shaft 462 and a bulbous tip 464 at the distal end of the shaft 462. The bulbous tip 464 is sized to engage the first bone tunnel in close fit sliding relationship. The resistance to insertion of the probe 460 is reduced as there is reduced or no friction between the wall of the bone tunnel and the shaft which is smaller than the tip. The length of the probe is significantly less than the depth of the first bone tunnel so that the bulbous tip will not intersect the projected retriever guide axis. On the upper side of the shaft member there is a generally triangular fin 466.

A retriever guide passage 468 is formed transversely through the shaft member and the fin along a retriever guide axis 470. The retriever guide axis 470 forms an angle with the shaft axis 458. When the head member of the guide is positioned near the greater tuberosity, the shaft axis is approximately level with the surgical site.

In use, a first bone tunnel 472 is formed and a suture loop is delivered into the first bone tunnel 472. For example, the suture loop may be delivered simultaneously with punching the tunnel or it may be delivered after the tunnel is formed. The probe 460 is inserted into the first bone tunnel and the retriever is advanced to the surgical site along the retriever guide passage to intersect the first bone tunnel and suture loop and retrieve the suture through a second bone tunnel.

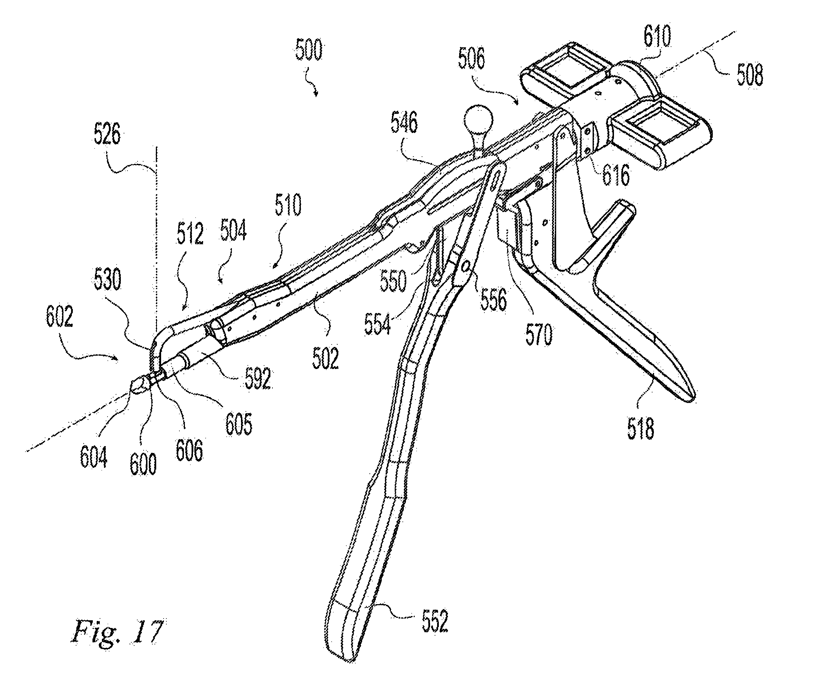

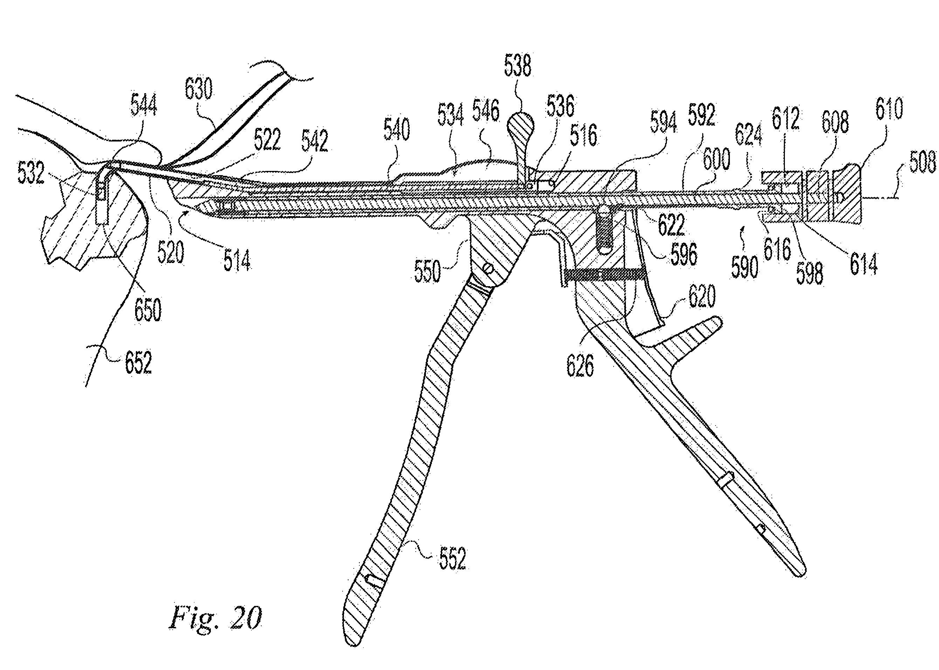

FIGS. 17-25 illustrate another illustrative example of a tunnel guide. The tunnel guide 500 includes a shaft member 502 extending between a distal end 504 and a proximal end 506 along a shaft axis 508. The distal end defines a distal head member 510 having an indexing feature in the form of a tubular probe 512 engageable with a first bone tunnel. The shaft member 502 includes a retriever guide passage 514 (FIG. 20) coaxial with the shaft axis 508 and extending between the proximal and distal ends 506, 504. The shaft member 502 includes a suture passer guide passage 516 parallel to the shaft axis 508 and extending from the distal end 504 toward the proximal end 506. A retriever assembly 590 (FIG. 20) is mounted to the shaft member 502 for translation within the retriever guide passage 514. A suture passer assembly 534 (FIG. 20) is mounted to the shaft member 502 for translation within a suture passer guide passage 516. A handle 518 extends transversely, downwardly from the shaft member and provides a grip for the tunnel guide. Each feature and assembly of the tunnel guide 500 will be described more fully below.

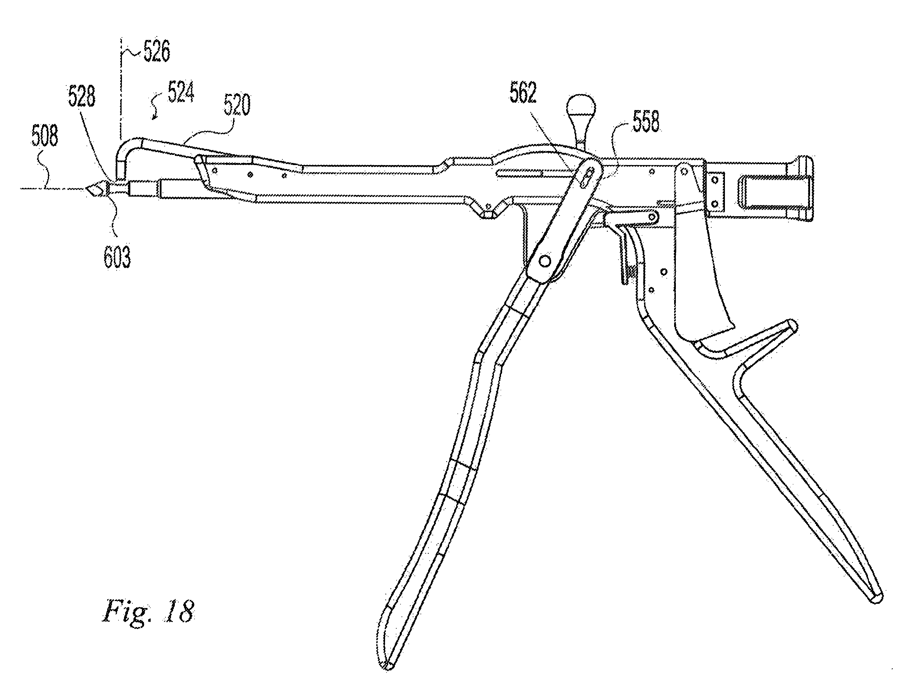

The tubular probe 512 indexing feature includes a bent tube 520 mounted to and extending distally from the head member 510. The tube 520 has an internal lumen 522 communicating with the suture passer guide passage 516 (Fig 20). The tube 520 diverges distally away from the shaft axis 508 and then bends back toward the shaft axis to form a hooked distal end 524 (FIG. 18) having a probe tip axis 526. The probe tip axis 526 (FIG 18) forms an angle with the shaft axis 508. In the illustrative example of FIGS. 14-22, the probe tip axis 526 is at an angle of approximately 90 degrees to the shaft axis 508 and has a probe tip exit opening 528. A suture slot 530 (FIG. 17) is formed in the distal end 524 of the probe to receive a suture portion 532 (FIG. 20) to position the suture portion 532 transverse to the internal lumen 522.

The suture passer assembly 534 is mounted for axial translation within the suture passer guide passage 516 and tube 520. The suture passer assembly 534 is releasably received by a carriage 536 and includes a suture passer having a handle 538 and a shaft 540 mounted to the handle 538. The suture passer shaft 540 has a flexible portion 542 ending in a tip 544. In the illustrative example of FIGS. 17-25, the flexible portion 542 and tip 544 are ribbon shaped to facilitate bending in one direction to follow the curvature of the bent tube 520. In the illustrative example of FIGS. 17-25, the suture passer assembly 534 is inserted into the suture passer guide passage 516 through a slot 546 (FIG. 17) and engaged with the carriage 536 such that the suture passers may be provided as easily replaceable modular units.

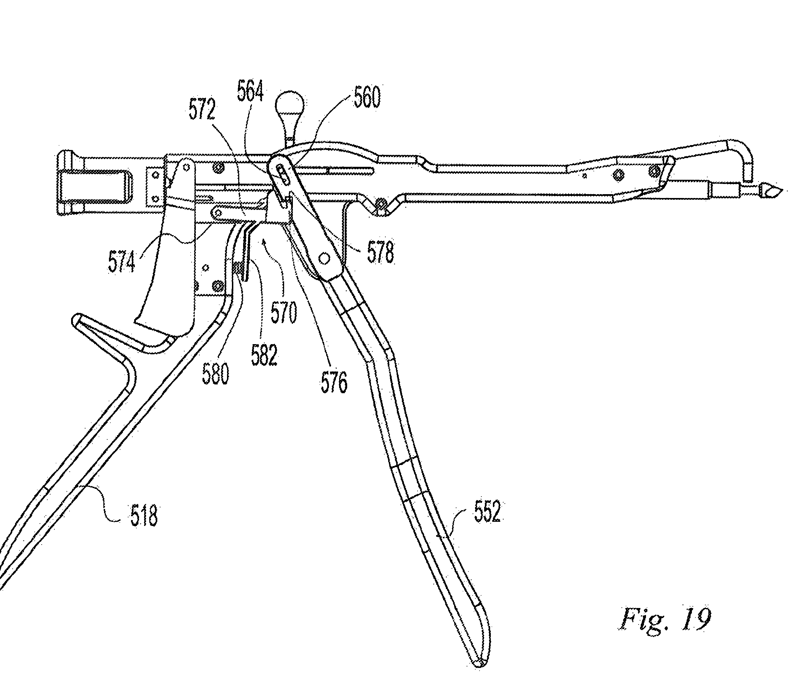

A tab 550 extends below the shaft member 502. A trigger 552 is mounted to the tab 550 for rotation. The trigger 552 includes a yoke 554 (FIG. 17) that straddles the tab and a pin 556 through the yoke and tab defines the rotation axis of the trigger. The ends 558, 560 of the yoke 554 (FIGS. 18 and 19) include longitudinal slots 562, 564, oriented radially from the center of the pin 556. A pin 566 through the carriage 536 is received in the slots 562, 564. As the trigger is rotated, the pin 566 follows the slots 562, 564 and converts the trigger rotation into linear translation of the carriage 536. As the carriage translates distally, it drives the suture passer assembly 534 distally causing the suture passer shaft 540 to bend as it follows the curve of the tube 520. Further motion of the suture passer assembly 534 causes the tip 544 to engage the suture portion 532 and pass it distally off of the tube 520 and away from the tube generally along the probe tip axis 526. Means for engaging the suture portion 532 by the tip 544 include one or more notches on the side of the tip or a notch located at the very distal portion of the tip. Such notches may be oriented in a manner such that the suture portion 532 is snared by the notch as the tip 544 moves distally and the suture portion 532 is released when the tip 544 is retracted. A trigger lock 570 (FIG. 19) may be provided to prevent inadvertent motion of the trigger 552 and premature deployment of the suture. In the illustrative example of FIGS. 17-25, the trigger lock includes an arm 572 (FIG. 19) mounted on a pivot 574 for rotation relative to the handle 518 and having a catch 576 spaced from the pivot that engages a slot 578 in the trigger yoke 554. A spring 580 presses on a catch lever 582 and biases the lock 570 into engagement with the trigger 552. Pressing on the catch lever 582 overcomes the spring pressure and rotates the arm 572 so that the catch disengages from the slot and the trigger is free to be rotated by pulling the trigger toward the handle.

The retriever assembly 590 (FIG. 20) includes a retriever sleeve 592 mounted for axial translation within the retriever guide passage 514 along the shaft axis 508. A detent 594 formed in the sleeve 592 is engageable with a spring biased plunger 596 mounted in the handle to give tactile feedback when the sleeve is retracted proximally to a predetermined axial position. The sleeve 592 includes a radial flange 598 at its proximal end. A retriever shaft 600 is mounted for axial translation within the retriever sleeve 592 along the shaft axis 508. The retriever shaft 600 includes a distal end 602 (FIG. 17) defining a sharpened distal tip 604 configured to penetrate bone and forming a shoulder 603 (FIG. 18). In the illustrative example of FIGS. 17-25, the tip 604 has an outer diameter equal to or slightly greater than a reduced diameter portion 605 (FIG. 17) of the distal end of the sleeve 592 such that the reduced diameter portion 605 of the sleeve can follow the tip 604 into a bone tunnel formed by impacting the tip 604 into a bone. A transverse aperture 606 is formed through the retriever shaft near the distal end 602 but proximal to the tip 604. The retriever shaft includes a proximal end 608 (FIG. 20) to which a knob 610 is mounted. Pushing the knob distally along the shaft axis 508 moves the retriever shaft 600 distally within the retriever sleeve 592. Pulling the knob proximally moves the retriever shaft 600 proximally within the retriever sleeve 592. An opening 612 in the distal end of the knob receives the flange 598 of the sleeve 592 in axial translating relationship. A back wall 614 or bottom of the opening 612 abuts the proximal end of the sleeve at the flange 598 and thus defines the distal most axial position of the retriever shaft 600 relative to the sleeve 592 when the knob 610 is pushed distally. Pins 616 mounted through the knob and transverse to the opening 612 abut the distal side of the flange 598 and thus define the proximal most axial position of the retriever shaft 600 relative to the sleeve 592 when the knob 610 is pulled proximally.

A sleeve lock 620 is mounted for rotation to the guide and includes a lip 622 that engages a shoulder 624 on the sleeve 592 to prevent the sleeve from moving proximally from its distal most position in the retriever guide passage 514. A spring 626 biases the sleeve lock 620 into engagement with the sleeve 592. Pressing the sleeve lock 620 overcomes the spring tension and pivots the sleeve lock 620 out of engagement with the sleeve 592 to release the sleeve.

FIGS. 20-25 illustrate the guide 500 in use. Referring to FIG. 20, the knob 610 is pulled proximally to position the retriever sleeve 592 and shaft 600 in their proximal most positions with the plunger 596 engaged with the detent 594 of the sleeve. A portion 532 of a suture 630 is placed in the slot 530 in the tip of the probe 512. A first bone tunnel 650 is formed in a bone 652. In the illustrative example of FIGS. 17-25, a vertical bone tunnel is formed in the humeral head below a torn rotator cuff. The probe 512 is inserted into the first bone tunnel and the angle of the guide 500 is adjusted to aim the shaft axis 508 at a desired entry location for a second bone tunnel. With the probe 512 inserted in the first bone tunnel 650, the guide 500 is indexed relative to the first bone tunnel.

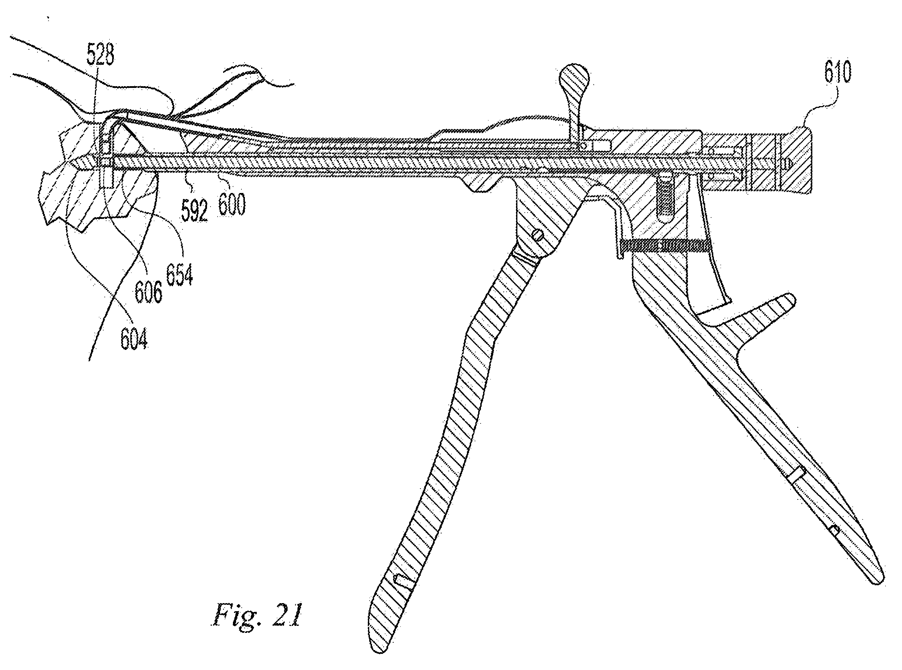

Referring to FIG. 21, the knob 610 is pushed distally until the tip 604 of the retriever shaft 600 is in contact with the bone. The knob is then impacted to drive the retriever shaft 600 and sleeve 592 into the bone and form a second bone tunnel 654. With the retriever shaft 600 and sleeve 592 driven to their proximal most position, the aperture 606 in the retriever shaft 600 is aligned with the exit opening 528 of the probe.

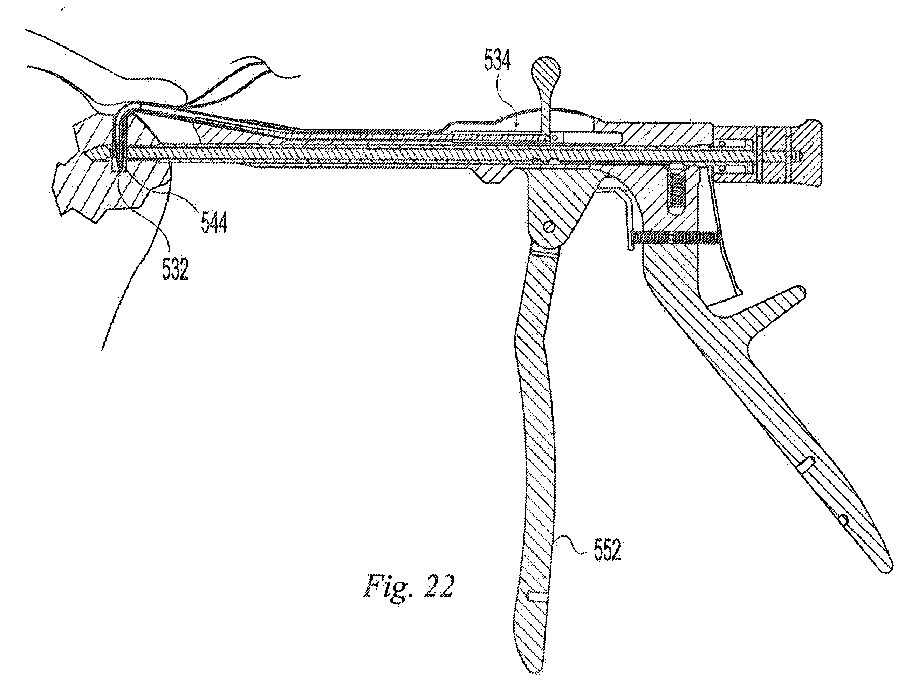

Referring to FIG. 22, the trigger 552 is released and pulled proximally to drive the suture passer assembly 534 distally. The tip 544 of the suture passer shaft 540 urges the suture portion 532 through the aperture 606.

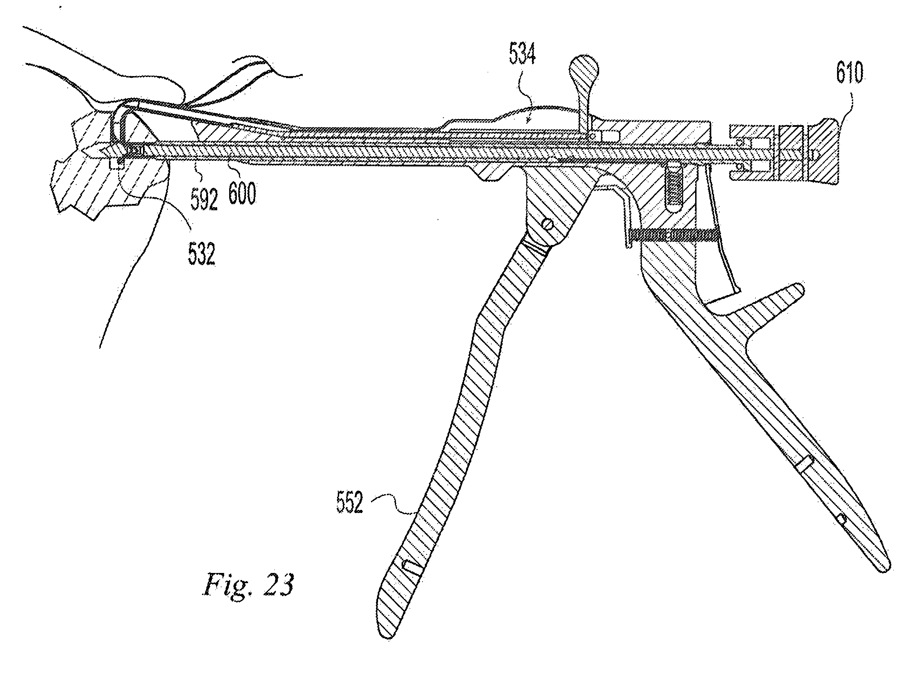

Referring to FIG. 23, the trigger 552 is moved distally to retract the suture passer assembly 534 leaving the suture portion 532 through the aperture 606. The knob 610 is pulled proximally to translate the retriever shaft 600 proximally relative to the sleeve 592 which retracts the aperture 606 into the distal end of the sleeve 592 and traps the suture between the distal end of the sleeve 592 and the shoulder 603 of the tip 604 of the retriever shaft.

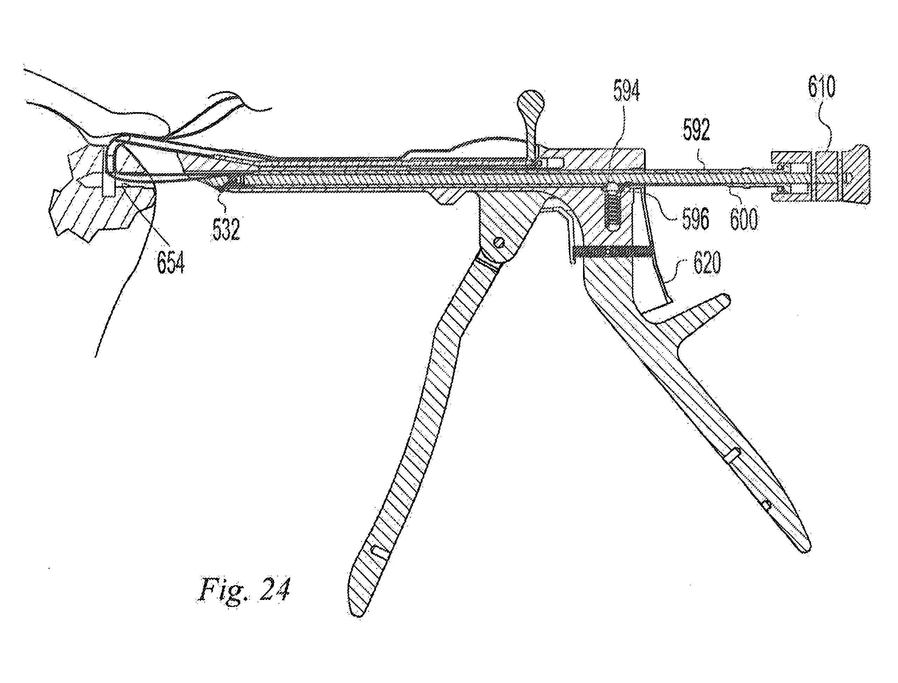

Referring to FIG. 24, the sleeve lock 620 is depressed to release the sleeve 592. The knob 610 is pulled proximally to pull the sleeve 592, retriever shaft 600, and suture portion 532 from the second bone tunnel 654. The knob 610 is pulled until the sleeve detent 594 engages the plunger 596 at which position the distal end of the sleeve 592 and retriever shaft 600 are withdrawn into the retriever guide passage 514 trapping the suture portion 532 within the distal end of the retriever guide passage 514.

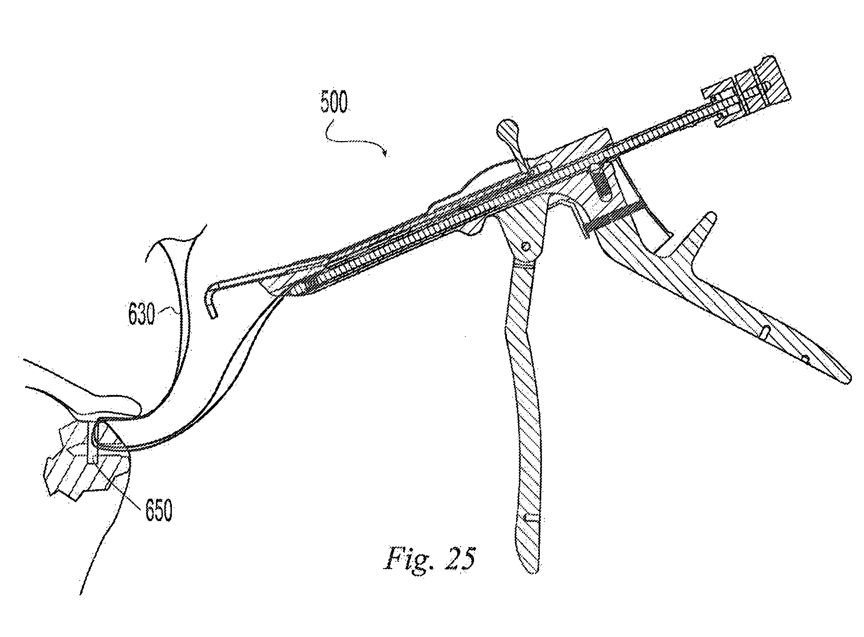

Referring to FIG. 25, the probe 512 is extracted from the first bone tunnel 650 and the guide 500 is pulled away from the surgical site to feed the suture 630 through the first and second bone tunnels. The suture 630 may now be used to affix the soft tissue to the bone in a transosseous manner.

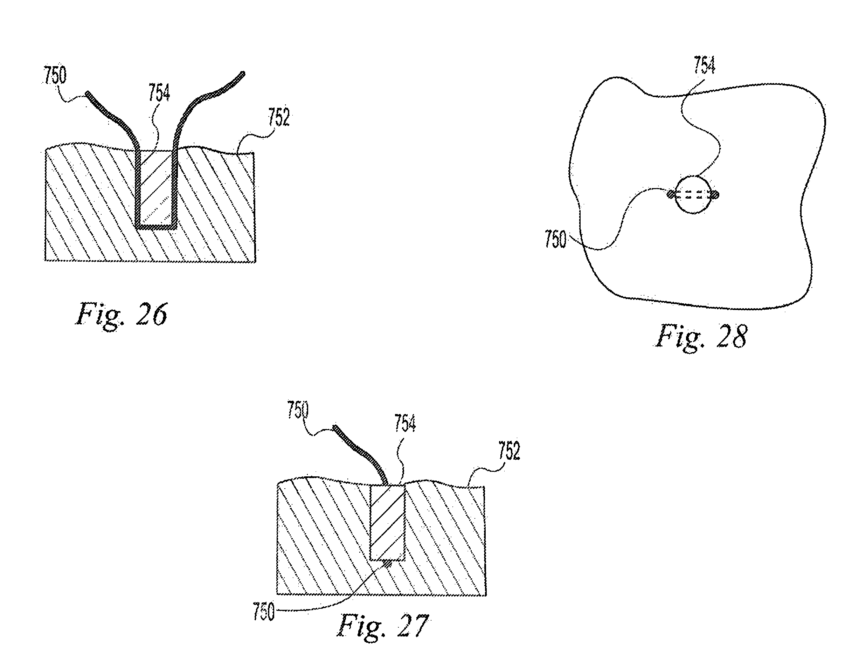

Instruments and a method have been described for placing a suture through a bone for a transosseous attachment technique. Aspects of the instruments and techniques may be used in a variety of ways. As an example, the suture punching technique may be used to efficiently place a suture into a bone anytime such a placement is needed and may eliminate the need for a separate drilling step. For example, the punch may be used to punch a suture into a bone after which the punch is removed and a plug is placed in the hole to trap the suture in the bone. For example, as shown in FIGS. 26-28, a suture 750 may be punched into a bone 752 and then a bone dowel 754 tapped in to form a completely tissue based suture-to-bone attachment. FIG. 27 is a cross sectional view. FIG. 24 is a cross sectional view 90 degrees from FIG. 26. FIG. 25 is a top plan view.

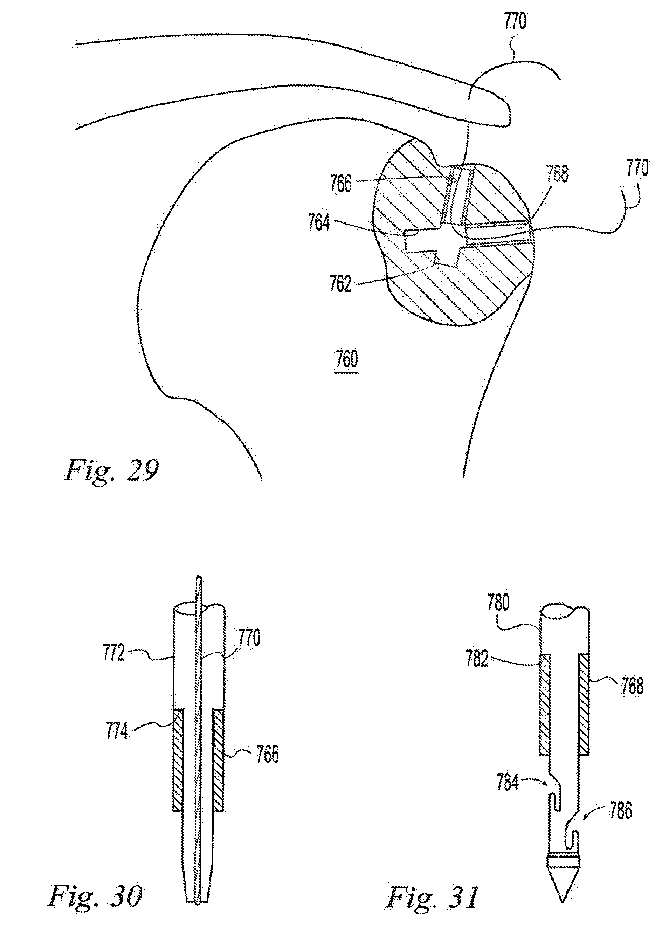

A bone tunnel may be reinforced by implanting a sleeve in the bone tunnel to prevent a suture from cutting into the bone when it is tensioned. FIG. 29 is a partial cross sectional view of a bone 760 with two intersecting bone tunnels 762, 764 formed such as with one of the previously described guides and techniques. Each tunnel has been reinforced with a sleeve implant 766, 768. Optionally, only one of the tunnels may be reinforced as desired. The sleeves 766, 768 may be inserted manually and a suture 770 threaded manually through them. Alternatively, the sleeves may be inserted simultaneously with the suture punch and/or retriever.

FIG. 30 is a partial cross sectional view illustrating a punch shaft 772 having a distal facing shoulder 774 formed on it. A sleeve 766 may be mounted on the shaft 772 abutting the shoulder 774 such that the punch, suture 770, and sleeve 766 are impacted into the bone 760 together. When the punch is withdrawn, the suture 770 and sleeve 766 are left behind with the sleeve 766 lining and reinforcing the bone tunnel 762.

FIG. 31 is a partial cross sectional view illustrating a retriever shaft 780 having a distal facing shoulder 782 formed on it proximal to notches 784, 786. A sleeve 768 may be mounted on the shaft 780 abutting the shoulder 782 such that the retriever and sleeve 768 are impacted into the bone 760 together. When the retriever is withdrawn to retrieve the suture, the sleeve 768 is left behind to line and reinforce the tunnel 764 and the suture is withdrawn through the sleeve 768.

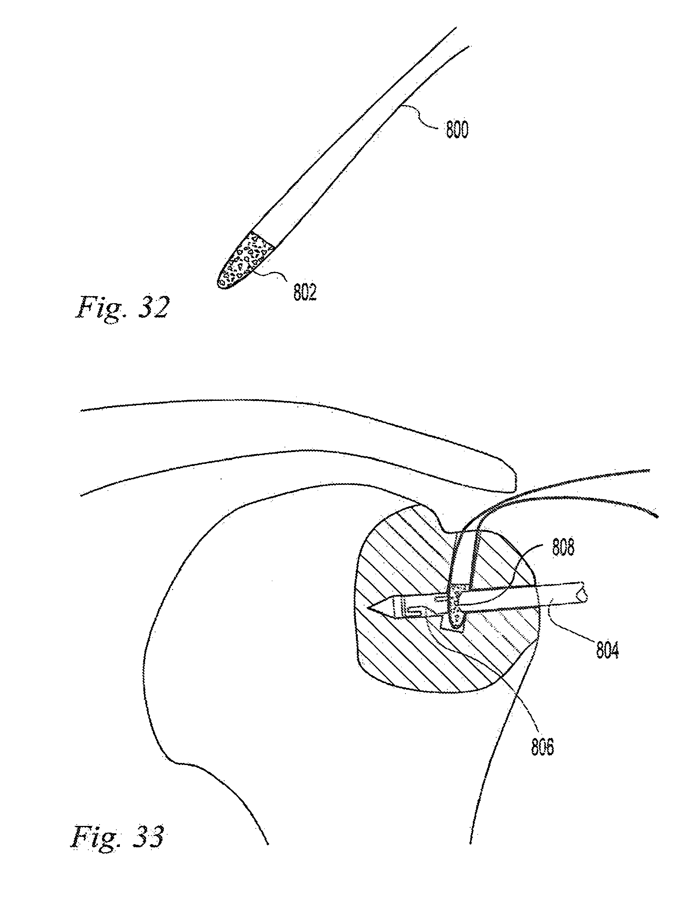

FIGS. 32 and 33 illustrate an alternative to a plain suture. A suture 800 has an expanded portion 802 that may be placed in the first bone tunnel to improve the efficiency by which the retriever 804 captures the suture. When the retriever 804 is inserted to intersect the first bone tunnel and suture, it passes through the expanded portion 802 of the suture. Upon withdrawal, the notch 806 in the retriever snags on the expanded portion 802 and thereby captures it. The expanded portion has a width dimension greater than the strand thickness of the suture. Because the expanded portion 802 presents a larger target for the retriever than a plain suture, it is more likely that the expanded portion will be captured regardless of which portion of the expanded portion the retriever passes through. As the retriever 804 passes through the expanded portion 802, it creates an opening 808 just large enough for the retriever to pass. As the retriever notch 806 reaches the expanded portion, this opening tends to collapse so that the edge of the opening falls within the notch 806 and is captured upon withdrawal of the retriever. The expanded portion 802 may include a membrane, enlarged braid, knitted fabric, woven fabric, parallel fibers, non-woven fabric, mesh, and/or other suitable portion larger than an individual suture strand. For example, a suture strand may be woven through a mesh, membrane, or fabric so that the material is captured at an intermediate portion of the suture. In another example, a portion of a suture may be unwound and teased apart to create an expanded portion.

While the foregoing illustrative examples have shown bone tunnels being formed by punching instruments into the bone, it is also within the scope of the invention to form bone tunnels by drilling, reaming, broaching, and/or any suitable tunnel forming process. The various guide instruments have illustrated different features for indexing the guide to the first bone tunnel in order to guide the formation of a second bone tunnel. The indexing features may include, but are not limited to, a tube, a passage, a slot, and a probe. It is contemplated, and within the scope of the invention, that the various features of the illustrative examples may be interchanged among the illustrative examples.

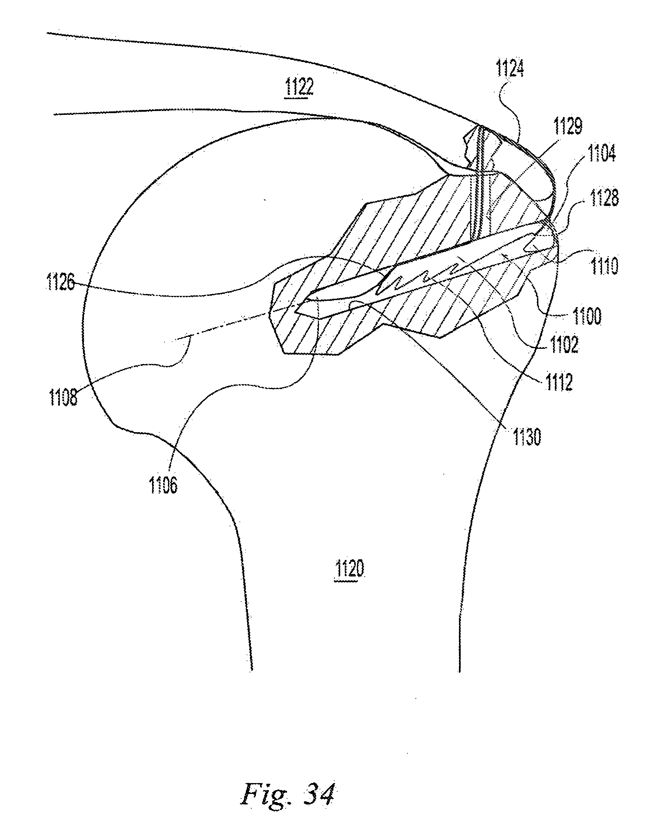

FIGS. 34-54 depict illustrative examples of suture anchoring implants.

Referring to FIG. 34 an osseous attachment device includes an implant 1100 having an elongated shaft 1102 extending from a proximal end 1104 to a distal end 1106 along an axis 1108. The shaft 1102 engages an elongated flexible strand to hold it relative to a bone. For example, FIG. 34 depicts a bone 1120 and soft tissue 1122 to be attached to the bone 1120 adjacent a skeletal joint; e.g. a proximal humerus and a portion of a rotator cuff. An elongate flexible strand 1124 such as a suture having first and second ends 1126, 1128 is passed through the soft tissue 1122 and into the bone 1120 at a desired attachment site. The implant 1100 is inserted into the bone 1120 to capture and retain the ends 1126, 1128 to hold the soft tissue 1122 adjacent the bone 1120.

For example, in a shoulder repair procedure, an elongate strand 1124 in the form of at least one closed suture loop may be passed through the soft tissue 1122 of the rotator cuff and the first end 1126 of the loop placed in the bone 1120 such as by placing it into a preformed tunnel or impacting it into the bone on a driver to simultaneously form a tunnel and insert the first end 1126. In the illustrative embodiment of FIG. 34, the first end 1126 is positioned in first tunnel 1129. A hook 1110 is formed adjacent the proximal end 1104 of the implant 1100 with a hook opening facing distally. The hook 1110 is engaged with the second end 1128 and inserted into the bone along a path intersecting the first end 1126. The implant 1100 and second end 1128 may for example be impacted directly into the bone to simultaneously form a second tunnel 1130 and insert the implant, or alternatively, they may be inserted into a preformed tunnel. In the illustrative embodiment of FIG. 34, one or more, optional barbs 1112 project from the shaft 1102 outwardly and distally. When the implant 1100 intersects the first end 1126, one or more of the barbs 1112 engage the first end 1126 such that when insertion of the implant 1100 is complete, the implant 1100 engages and secures both ends of the elongate strand 1124 to retain the elongate strand in the bone 1120 and secure the soft tissue 1122 with the first end being secured distally and the second end being secured proximally. Alternatively, the barbs 1112 may be omitted and the shaft 1102 alone pass through the suture loop to constrain it within the bone. With the use of a preformed loop, the soft tissue attachment is accomplished without the need for the user to tie any knots.

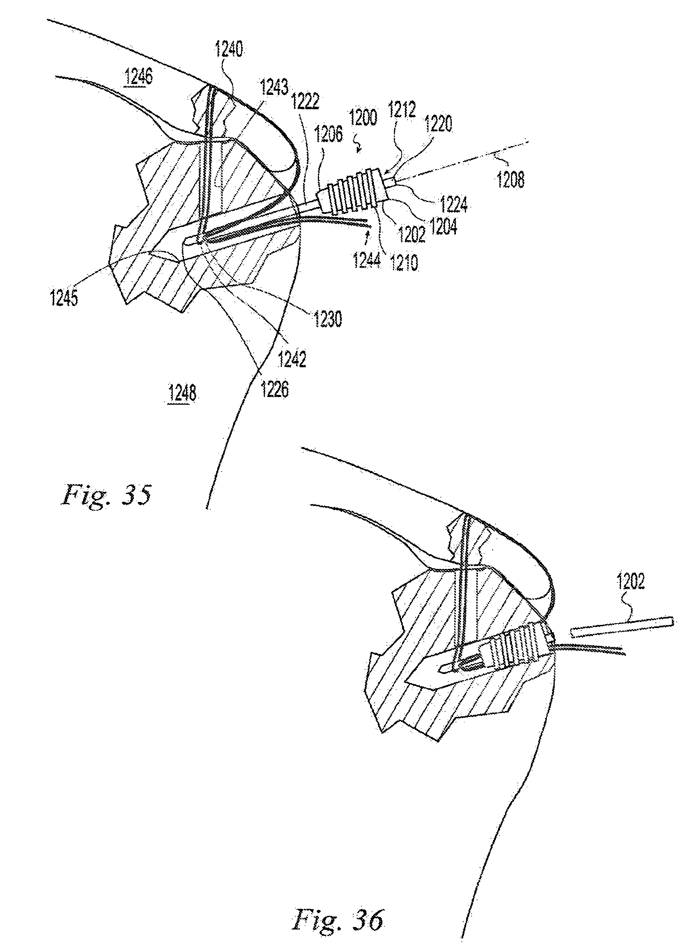

Referring to FIGS. 35 and 36, an osseous attachment device includes a locking implant 1200 and an elongated member 1220. In the illustrative embodiment of FIGS. 35 and 36, the implant 1200 is in the form of an interference screw. The implant 1200 includes an elongated tapered body 1202 extending from a wider, proximal end 1204 to a narrower, distal end 1206 along an axis 1208. A spiral thread 1210 is formed on the exterior of the body 1202 and the body includes an axial through passage 1212. In the illustrative embodiment of FIG. 35, the separate elongated member 1220 is in the form of a suture carrier that includes an elongated shaft 1222 extending from a proximal end 1224 to a distal end 1226. The distal end 1226 may be tapered or otherwise sharpened to ease insertion into bone. A transverse opening 1230 is formed through the shaft near the distal end 1226. An optional groove or reduced diameter region may be provided proximal of the opening 1230 to ease in cutting or breaking the elongated member 1220 to a desired length. The passage 1212 of the implant and shaft 1222 of the elongated member are sized for axial translating engagement. The elongated member 1220 is used to capture the ends of an elongated flexible strand and the implant 1200 is used to lock the elongated flexible strand to hold the elongated flexible strand adjacent to a bone.

For example, in a shoulder repair procedure, an elongate strand 1240 in the form of at least one suture defining a first end 1242 in the form of a loop, or bight, and having second ends 1244, may be passed through the soft tissue 1246 of the rotator cuff. The first end 1242 is inserted into the bone 1248 such as by placing it into a preformed tunnel 1243 or impacting the elongate strand 1240 into the bone on a driver to simultaneously form a first tunnel and insert the first end 1242. The second ends 1244 are passed through the transverse opening 1230 of the elongated member 1220 and the elongated member 1220 and second ends 1244 are inserted into the bone along a path that intersects the first end 1242. The elongated member 1220 and second ends 1244 may for example be impacted directly into the bone to simultaneously form a second tunnel and insert elongated member 1220 and second ends 1244, or alternatively, they may be inserted into a preformed second tunnel 1245. When the elongated member 1220 intersects the first end 1242, the distal end 1226 of the elongated member 1220 captures the first end 1242 distally and prevents it from being withdrawn upwardly through the bone such that the first end 1242 is retained distally in the bone. The second ends 1244 may then be pulled to feed slack through the transverse opening 1230 and tension the elongated strand 1240 and approximate the soft tissue to the bone. The locking implant 1200 is then engaged with the proximal end of the elongated member 1220 and advanced into the bone 1248. The locking implant 1200 presses the elongated strand 1240 against the bone in an interference engagement to lock the second ends 1244 in the second tunnel 1245. The locking implant also prevents the elongated member 1220 from exiting the second tunnel 1245 thus the locking implant locks both ends of the elongated strand 1240 relative to the bone 1248 and secures the soft tissue 1246. The soft tissue attachment is accomplished without the need for the user to tie any knots.

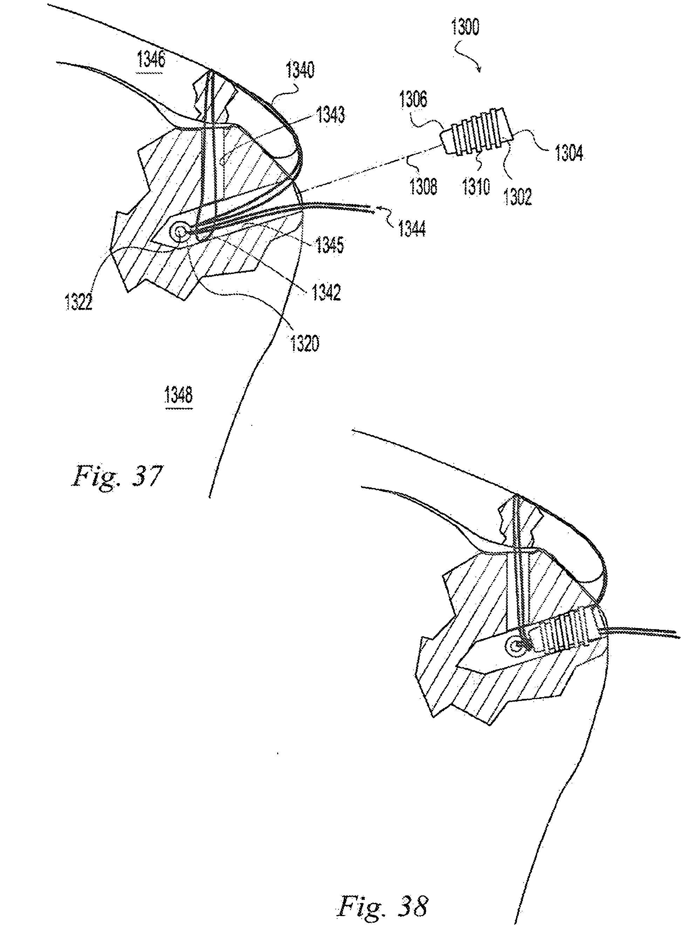

Referring to FIGS. 37 and 38, an osseous attachment device includes a locking implant 1300 and a suture carrier 1320 similar to that of FIGS. 35 and 36. In the illustrative embodiment of FIGS. 37 and 38, the implant 1300 is in the form of an interference screw having a cylindrical body 1302 extending from a proximal end 1304 to a distal end 1306 along an axis 1308. A spiral thread 1310 is formed on the exterior of the body 1302. In the illustrative embodiment of FIGS. 37 and 38, the separate suture carrier 1320 is in the form of a ring having an aperture 1322. The suture carrier 1320 is used to capture the ends of an elongated flexible strand and the locking implant 1300 is used to lock the elongated flexible strand to hold the elongated flexible strand adjacent to a bone. While a suture carrier has been shown in the form of a ring it may have other forms such as a sphere, rod, or other suitable shape that can receive a suture in sliding relationship.

For example, in a shoulder repair procedure, as shown in FIGS. 37 and 38, an elongate strand 1340 in the form of at least one suture defining a first end 1342 in the form of a loop, or bight, and having second ends 1344, may be passed through the soft tissue 1346 of the rotator cuff. The first end 1342 is inserted into the bone 1348 such as by placing it into a preformed first tunnel 1343 or impacting the elongate strand 1340 into the bone on a driver to simultaneously form a tunnel and insert the first end 1342. The second ends 1344 are passed through the aperture 1322 of the suture carrier 1320 and the suture carrier 1320 and second ends 1344 are inserted into the bone along a path that intersects the first end 1342. The suture carrier 1320 and second ends 1344 are passed through the loop of the first end 1342. Applying tension to the elongated strand 1340 causes the loop of the first end 1342 to close around the second ends 1344 and trap the suture carrier 1320 in the bone such that the ends 1342, 1344 are retained in the bone. Further pulling on the second end 1344 causes slack to feed through the suture carrier and tension the strand 1340 to approximate the soft tissue to the bone. The locking implant 1300 is then advanced into the bone 1348. The locking implant 1300 presses the elongated strand 1340 against the bone in an interference engagement to lock the elongated strand 1340 relative to the bone 1348 and secure the soft tissue 1346. The soft tissue attachment is accomplished without the need for the user to tie any knots.

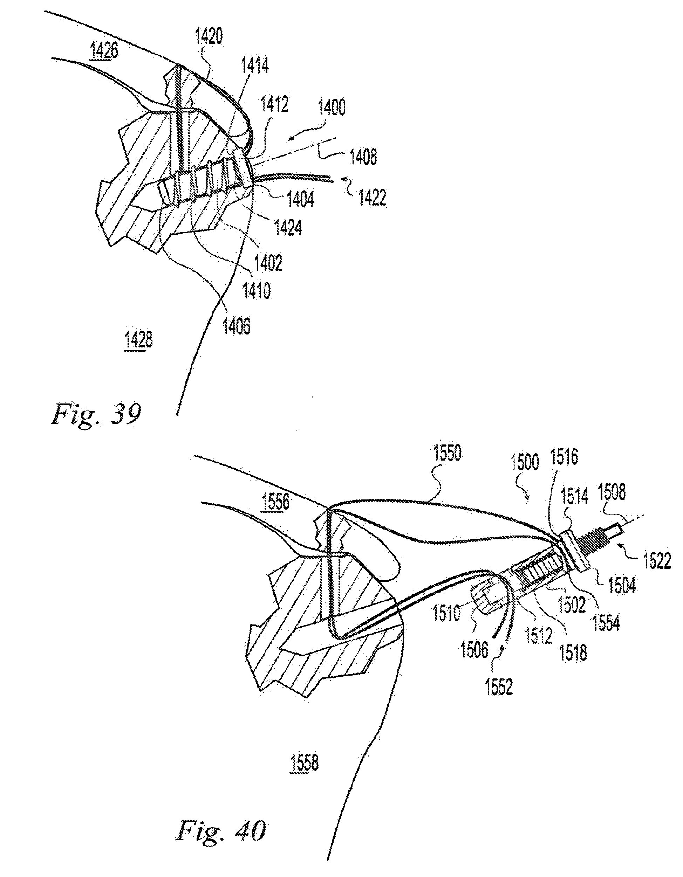

Referring to FIG. 39, an osseous attachment device includes an implant 1400. In the illustrative embodiment of FIG. 39, the implant 1400 is in the form of an interference screw. The implant 1400 includes an elongated body 1402 extending from a proximal end 1404 to a distal end 1406 along an axis 1408. A spiral thread 1410 is formed on the exterior of the body 1402. A head 1412 is formed near the proximal end 1404 and defines a distally facing shoulder 1414 at the junction of the head 1412 and body 1402. The implant 1400 is used to capture both ends of an elongated flexible strand 1420 and hold the elongated flexible strand 1420 adjacent to a bone.

For example, in a shoulder repair procedure, as shown in FIG. 39, an elongated strand 1420 in the form of at least one suture having first ends 1422 and a second end 1424 defining a loop, may be passed through the soft tissue 1426 of the rotator cuff. The first end 1422 is inserted through the bone 1428. The second end 1424 is engaged with the distal end 1406 of the implant 1400. The first end 1422 may be tensioned to remove slack and press the soft tissue against the bone. The distal end 1406 of the implant 1400 may be braced against the bone or engaged with the bone tunnel to facilitate tensioning the strand 1420. The implant 1400 is then driven into the bone to lock the ends 1422, 1424 relative to the bone. The second end 1424 is trapped beneath the head 1412 adjacent the shoulder 1414 of the implant 1400 and the first end is trapped between the thread 1410 and bone 1428.

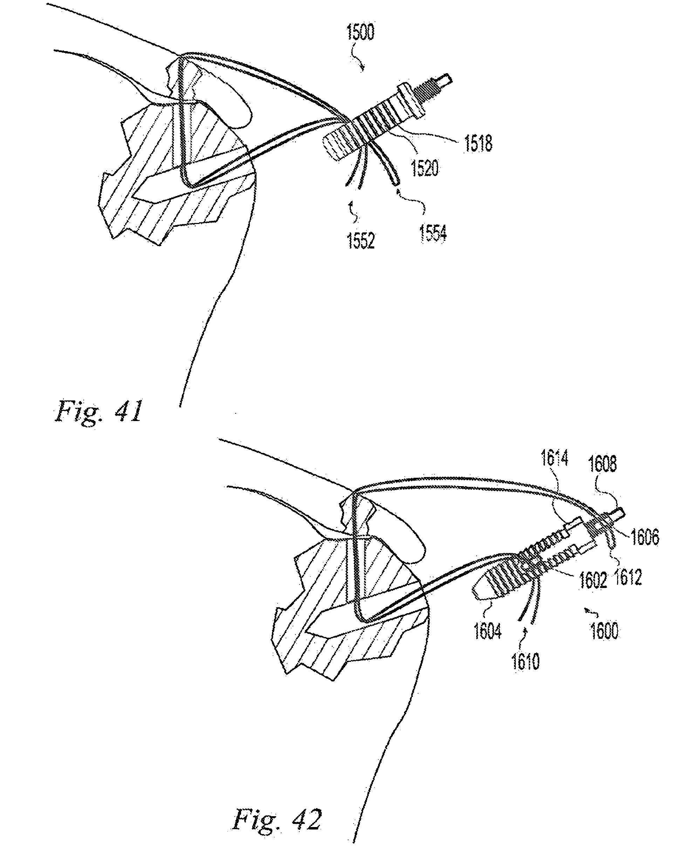

Referring to FIGS. 40 and 41, an osseous attachment device includes an implant 1500. The implant includes an elongated body 1502 extending from a proximal end 1504 to a distal end 1506 along an axis 1508. An axial bore 1510 extends into the body 1502 proximally to distally. A transverse body aperture 1512 extends through the body and intersects the axial bore 1510. A head 1514 is formed near the proximal end 1504 and defines a distally facing shoulder 1516 at the junction of the head 1514 and body 1502. The head 1514 is interrupted by opposed grooves aligned with the aperture 1512. Opposed flat surfaces 1518 on the exterior of the body are aligned with the grooves and the aperture 1512 and the grooves and flat surfaces 1518 provide clearance to allow a suture to slide between the body 1502 and a bone tunnel wall. The exterior of the body further includes annular projections 1520 on opposite sides of the body 1502 between the flat surfaces 1518. The annular projections engage a bone tunnel wall to retain the implant 1500 in the bone tunnel. The axial bore 1510 is threaded proximally and receives a plunger 1522 in axial threaded engagement such that the plunger is responsive to rotation to move between a first position in which the plunger 1522 distal end is substantially not overlapping the transverse body aperture 1512 and a second position in which the plunger 1522 overlaps at least a portion of the transverse body aperture 1512.

The implant 1500 is used to capture both ends of a strand and hold the strand adjacent to a bone. For example, in a shoulder repair procedure, as shown in FIG. 40, an elongated strand 1550 in the form of at least one suture having a first end 1552 and a second end 1554 defining a loop, may be passed through the soft tissue 1556 of the rotator cuff. The first end 1552 is inserted through the bone 1558. The second end 1554 is looped around the body 1502 such as by inserting the implant 1500 through the loop of the second end 1554 until the loop comes to rest against the shoulder 1516. The first end 1552 is passed through the aperture 1512. The first end is then passed along the flat surface 1518 and through the groove in the head. The implant 1500 is inserted into the bone until the shoulder 1516 abuts the bone 1558. The first end 1552 of the strand 1550 is tensioned to remove slack and press the soft tissue against the bone. The plunger 1522 is advanced toward the transverse aperture 1512 until the distal end of the plunger 1522 traps the second end 1554 of the strand in the axial bore 1510 such that the first end 1552 is fixed distally in the aperture 1512 and the second end 1554 is trapped proximally under the shoulder 1516.

FIG. 41 illustrates an alternative method of using the implant 1500. In the illustrative method of FIG. 41, both ends 1552, 1554 of the suture are passed through the transverse aperture 1512 of the implant 1500 and along the flat surface 1518 and through the groove in the head. The implant 1500 is inserted into the bone until the shoulder 1516 abuts the bone 1558. The ends 1552, 1554 of the strand 1550 are tensioned to remove slack and press the soft tissue against the bone. The plunger 1522 is advanced toward the transverse aperture 1512 until the distal end of the plunger 1522 traps the ends 1552, 1554 of the strand distally in the aperture 1512 intersecting the axial bore.

Referring to FIG 42, an osseous attachment device includes an implant 1600 similar to that of FIG. 40 and 41 except that the implant 1600 of FIG. 42 includes a transverse aperture 1602 through the implant body 1604 and a transverse aperture 1606 through the plunger 1608 and the plunger 1608 is advanced by pressing it into the body 1604 rather than by threading. In this example, the first end 1610 of the suture strand is passed through the body aperture 1602 and the second end 1612 is passed through the plunger aperture 1606. When the plunger 1608 is advanced in the body 1604, the distal end of the plunger traps the first end 1610 in the body aperture 1602 and the plunger aperture 1606 and head 1614 trap the second end 1612. The relationship between the plunger length and positions of the apertures 1602, 1606 may be adjusted to provide for simultaneous locking of the suture ends, distal locking of the first suture end 1610 before proximal locking of the second suture end 1612, or proximal locking of the second suture end 1612 before distal locking of the first suture end 1610.

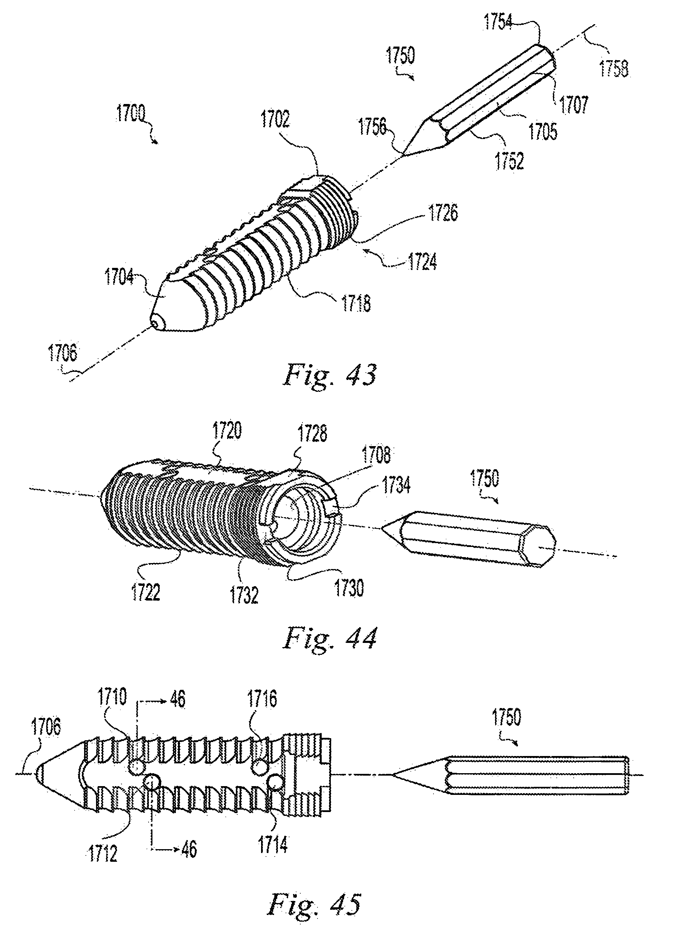

Referring to FIGS. 43-48, an osseous attachment device according to the present invention includes an elongate implant body 1700 and a plunger or piston 1750 receivable in the body 1700. The implant body extends from a proximal end 1702 to a distal end 1704 along an axis 1706. An axial passage 1708 extends into the body proximally to distally along the axis 1706. First and second transverse apertures 1710, 1712, forming a distal aperture pair, extend through the body 1700 distally and intersect the axial passage 1708. The apertures 1710, 1712 are offset toward opposite sides of the axis 1706 and the second aperture 1712 is offset proximally from the first aperture 1710. Third and fourth transverse apertures 1714, 1716, forming a proximal aperture pair, extend through the body 1700 proximally and intersect the axial passage 1708. The apertures 1714, 1716 are offset toward opposite sides of the axis 1706 and the fourth aperture 1716 is offset distally from the third aperture 1714. The body 1700 has radially extending ridges 1718 that taper distally to aid in retaining the body in a tunnel. Opposed flats 1720, 1722 extend along opposite sides of the body 1700 adjacent the apertures 1710, 1712, 1714, 1716 to provide clearance for suture ends extending alongside the body 1700. A head 1724 formed near the proximal end extends radially outwardly beyond the body diameter and includes radially extending ridges 1726. Opposed flats 1728, 1730 extend along opposite sides of the head 1724 in circumferential alignment with the body flats 1720, 1722 and apertures 1710, 1712, 1714, 1716 but spaced radially outwardly from the axis 1706 farther than the flats 1720 and 1722. Alignment slots 1732, 1734 are formed on the distal end of the head to provide a rotational alignment keyway for a driver (not shown). The distal end 1704 of the body tapers distally to ease insertion into a tunnel.

The plunger 1750 includes an elongated body 1752 extending from a proximal end 1754 to a distal end 1756 along an axis 1758. The distal end of the plunger tapers distally to ease insertion into the body 1700 and separate suture strands as will be more fully described below. The plunger 1750 has faceted sides 1705 defining elongated vertices, or ridges 1707, at the intersection of adjacent facets. The plunger 1750 is receivable in the passage 1708 in axial translating relationship.

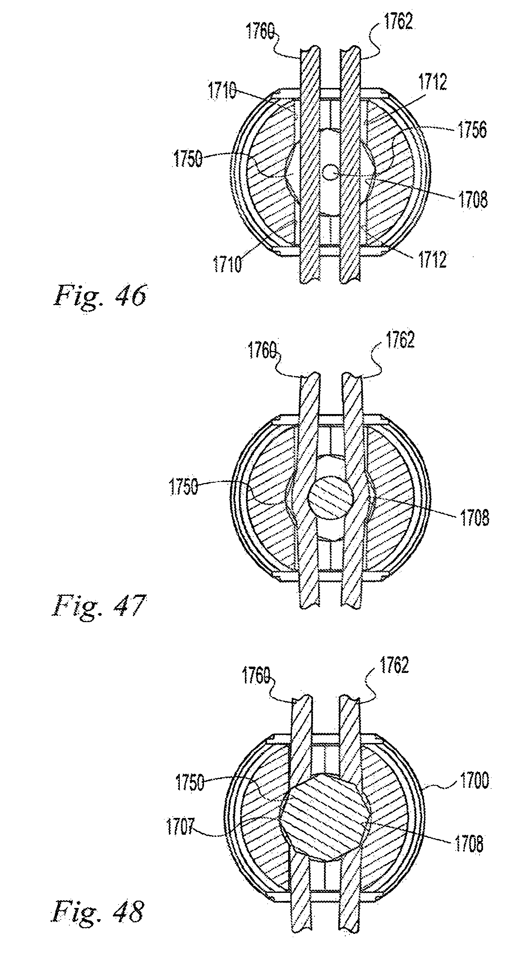

One or more suture strands may be passed through the apertures 1710, 1712, 1714, 1716 and locked with the plunger 1750. The plunger can lock any number of suture strands passing through any number of the apertures. Referring to FIG. 46, a suture strand 1760, 1762 has been passed through each of apertures 1710 and 1712. The plunger 1750 has been advanced distally into the passage 1708 until the distal end 1756 of the plunger 1750 is just short of touching the suture strands 1760, 1762. As can be seen in FIG. 46, since the apertures 1710, 1712 are offset outwardly from the axis 1706, the tapered distal end 1756 of the plunger, which is coaxial with axis 1706, is directed between the strands 1760, 1762.

Referring to FIG. 47, the plunger has been advanced further distally and the distal end 1756 has moved between the strands 1760, 1762 and begun pressing them outwardly toward the side wall of passage 1708.

Referring to FIG. 48, the plunger 1750 has been advanced fully into the passage 1708 and tightly compresses the strands 1760, 1762 between the plunger sides and passage 1708 such that the suture strands are locked firmly relative to the body 1700. The plunger 1750 presses the strands sideways into the sidewall of passage 1708 and the suture strands are highly compressed by the ridges 1707. Since the ridges 1707 are able to slide smoothly over the sutures while compressing them, the advancing plunger 1750 locks the suture strands without dragging the sutures axially along the passage 1708 and therefore the suture strands are locked with little or no change in the suture tension.

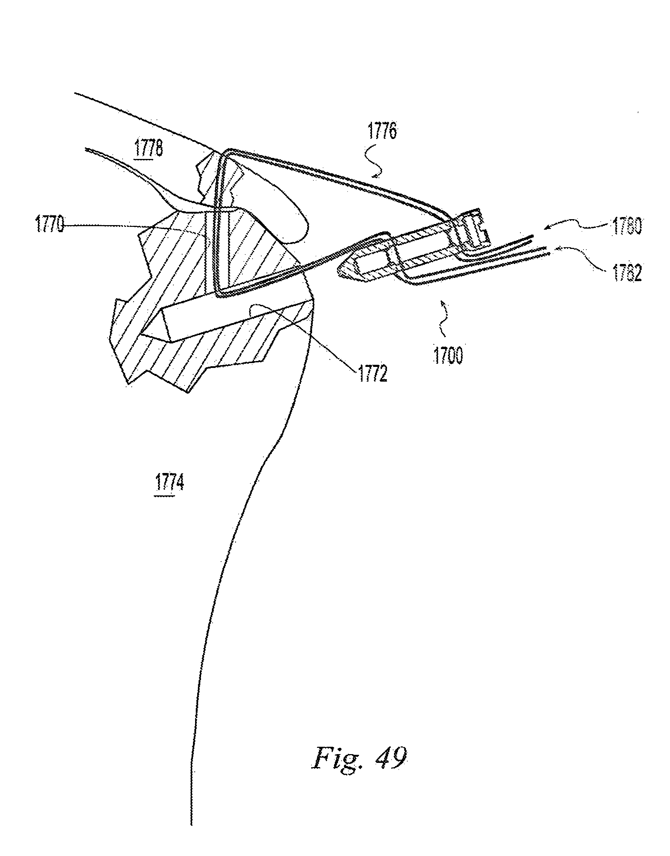

FIGS. 49-53 depict an illustrative example of a method of using the implant of FIGS. 43-48 in a surgical procedure to secure a portion of a rotator cuff to a proximal humerus using knotless transosseous suture fixation.

Referring to FIG. 49, first and second intersecting bone tunnels 1770, 1772 have been formed in the head of a humeral bone 1774 of a shoulder joint. Suture strands 1776 have been passed through the bone tunnels with first ends 1780 exiting superiorly from the first bone tunnel and passing through a portion of the rotator cuff 1778 and second ends 1782 exiting laterally from the second bone tunnel 1772. The first ends 1780 have been passed through the proximal apertures 1714, 1716 of the implant body 1700 and the second ends 1782 have been passed through the distal apertures 1710, 1712. The second bone tunnel 1772 is sized to be a press fit with the ridges 1718 of the body 1700.

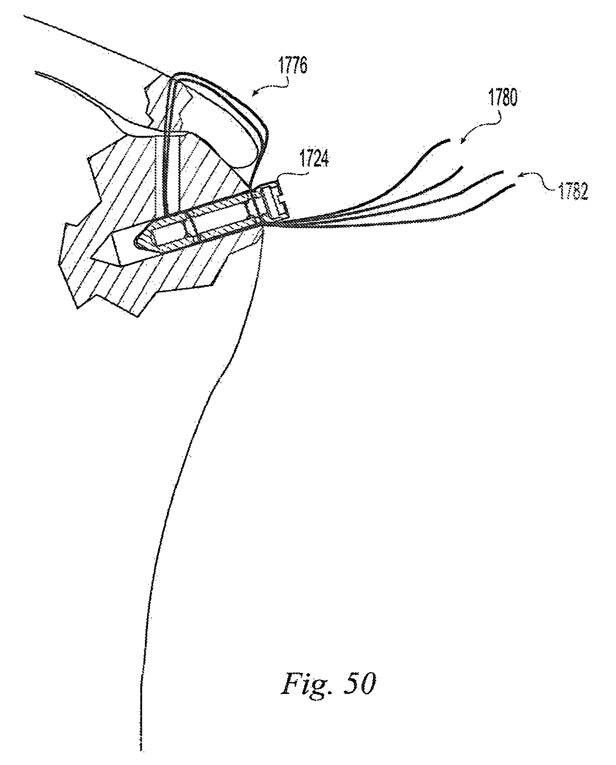

Referring to FIG. 50, the body 1700 has been inserted into the second bone tunnel 1772 up to the base of the head 1724. In this position, the suture ends 1780, 1782 may be pulled to remove slack from the suture strands 1776 and the strands will slide easily through the bone tunnels and implant body 1700.

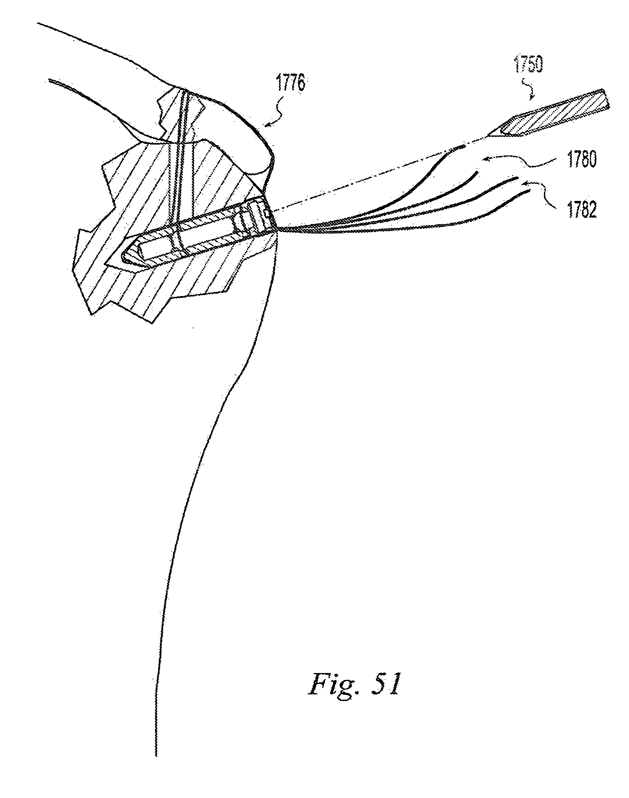

Referring to FIG. 51, the body 1700 has been further inserted into the second bone tunnel 1772 so that the head is flush with the bone and the head compresses the suture strands between the head and bone. Since the head 1724 extends radially outwardly farther than the body 1700, driving the head into the bone will compress the suture strands as shown in a provisionally locked state. In this state, the sutures will not slip easily but a user can supply sufficient force to the ends 1780, 1782 to overcome the frictional provisional lock and perform a final tensioning of the suture strands 1776. The plunger 1750 is shown aligned with the passage 1708 ready to be inserted after final tensioning of the suture strands 1776 is completed.

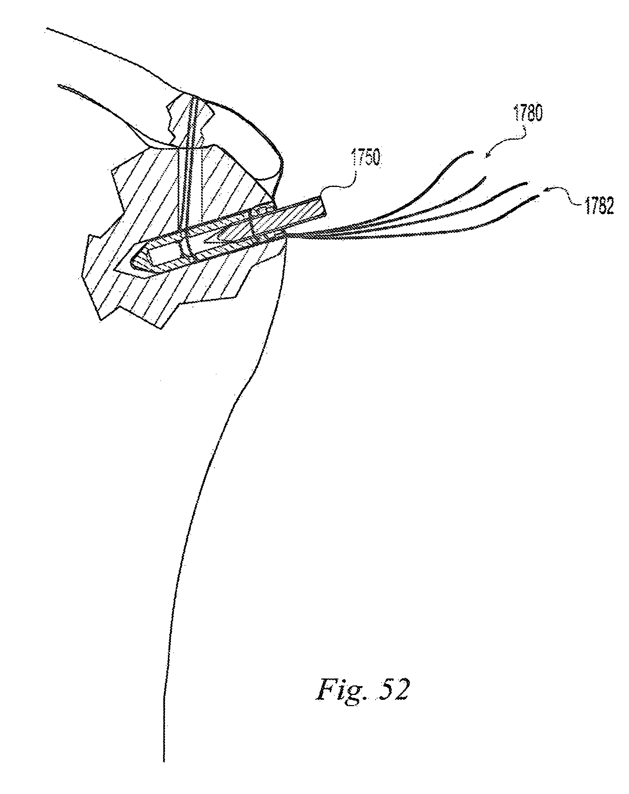

Referring to FIG. 52, the plunger 1750 has been inserted partway into the passage 1708 so that the suture ends 1780 passing through the proximal aperture pair are locked but the suture ends 1782 passing through the distal aperture pair can still be tensioned if desired. In this way the plunger 1750 provides a sequential locking action relative to the proximal and distal apertures.

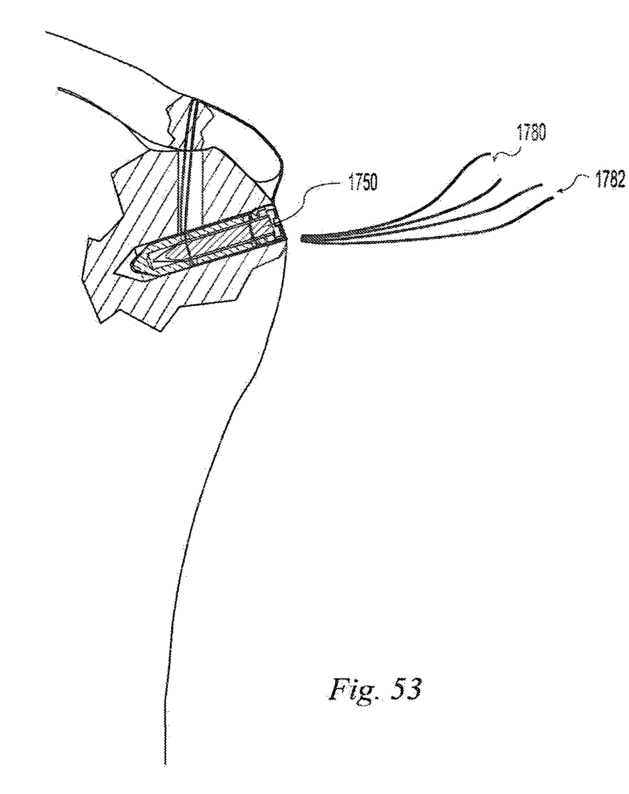

Referring to FIG. 53, the plunger 1750 has been fully seated locking all of the suture strands and the loose suture ends have been cut off flush with the bone.

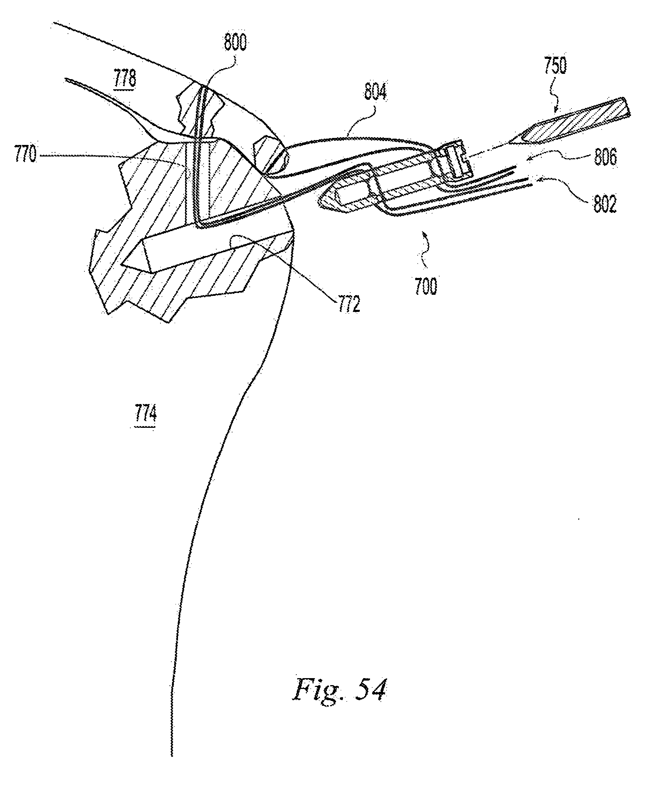

FIG. 54 illustrates an alternative method of using the implant 1700 in which separate suture strands are passed through separate portions of a soft tissue and the loose ends of each strand are secured using the distal and proximal pairs of apertures respectively. A first suture strand 1800 is attached to the rotator cuff 1778 such as by way of a mattress stitch or other suitable stitch. The ends 1802 of the first suture strand 1800 have been passed through the distal apertures 1710, 1712 of the implant body 1700. A second suture strand 804 is attached to the rotator cuff 1778 such as by way of a mattress stitch or other suitable stitch. The ends 806 of the second suture strand 804 have been passed through the proximal apertures 1714, 1716 of the implant body 1700. The implant body 1700 is inserted into the bone tunnel 1772 and the sutures tensioned and secured as describe in the previous illustrative example.

The foregoing examples have illustrated various embodiments of devices and methods useful to attach an elongated strand to a bone by forming a tunnel through the bone, passing the strand through the bone, and then capturing both ends of the strand with a single implant. The embodiments have been illustrated in use to repair a rotator cuff of a shoulder joint but it will be understood that the devices and methods are applicable at other surgical sites to attach other implant and tissues to bone. For example, the devices and methods may be used to attach sutures, tendons, ligaments, cables, implant anchor portions, and/or other objects to bone at surgical locations throughout a patient's body. The devices and methods have been shown in use with first and second transverse, linear, intersecting bone tunnels. However, the devices may be used with single linear tunnels through a bone, curved tunnels, three or more intersecting bone tunnels, and/or other bone tunnel configurations in which it is desired to lock with a single device multiple suture ends.

In the illustrative examples, anchors have been shown securing suture portions at various locations of the anchor. For example, some of the examples have described or depicted fixation at a proximal portion of the anchor and/or at a distal portion of the anchor. The proximal and distal portions of the anchor may refer to distinct proximal and distal ends of the anchor. The proximal and distal portions may refer to relative regions of the anchor such as the proximal one half and distal one half of the anchor, the proximal one third and distal two thirds of the anchor, the proximal two thirds and distal one third or the anchor, or some other fractional part referring to distinct relative zones.

The different illustrative examples have been shown with various forms of bone fixation including threads and annular ridges of varying size and shape. These different forms of fixation may be interchanged within the scope of the invention, which is defined by the claims. For example, where ridges are shown, threads may be substituted and where threads are shown, ridges may be substituted. Any other form of fixation known in the art may also be substituted including but not limited to a smooth press fit.

Some of the illustrative examples have included a plunger receivable within an implant body to lock a suture portion relative to the implant body. In these illustrative examples, the plunger has been shown as engaging the implant body for axial translation by threading, ratcheting, or smooth press fitting into the implant body. These engagement arrangements may be interchanged among the different plunger embodiments. Furthermore, other features for retaining the plunger within the implant body may be incorporated on the plunger and/or within the implant body including ridges, grooves, bumps, surface textures, and/or other retaining features. Furthermore, while the illustrative examples have depicted plungers that are moveable from a proximal position to a distal position in which the suture portion is secured, the plunger may also be moveable from a distal position to a proximal position in which the suture portion is secured. For example, a plunger may be disposed in the implant body distal to a transverse opening and be pulled proximally to secure a suture in the transverse opening.

## Claims

1. A suture anchor comprising:
an elongated body (1700) having a proximal end (1702) and a distal end (1704), the body having an outer surface with radially extending bone engaging ridges (1718, 1726) formed thereon, the body having a longitudinal passageway (1708) extending from the proximal end toward the distal end and defining a longitudinal passageway axis (1706) and an inner wall, and a solid plunger (1750) having a proximal end and a distal end, **characterized in that** the body has a first transverse suture receiving passageway extending through the body (1714, 1716) nearer the proximal end and intersecting the longitudinal passageway and a second transverse suture receiving passageway (1710, 1712) extending through the body nearer the distal end and intersecting the longitudinal passageway, the first and second suture receiving passageways being spaced apart along the longitudinal passageway axis; and the solid plunger is
receivable within the longitudinal passageway in axial translating relationship between a first position in which the distal end of the plunger is proximal to the first transverse suture receiving passageway and a second position in which the distal end of the plunger is distal to the second transverse suture receiving passageway and the proximal end of the plunger is proximal to the first transverse suture receiving passageway, the plunger being operable in the second position to compress suture portions received in each of the first and second transverse openings radially between the plunger and the inner wall of the longitudinal passageway.

2. The suture anchor of claim 1 further comprising third (1710, 1712) and fourth (1714, 1716) transverse suture receiving passageways, each extending through the body and intersecting the longitudinal passageway, the first and third transverse suture receiving passageways forming a distal pair of passageways offset relative to one another toward opposite sides of the longitudinal passageway axis, the second and fourth transverse suture receiving passageways forming a proximal pair of passageways offset relative to one another toward opposite sides of the longitudinal passageway axis.

3. The suture anchor of claim 2 wherein the distal end (1756) of the plunger tapers to a narrow distal tip contained within the longitudinal passageway in the second position.

4. The suture anchor of claim 1 wherein the plunger comprises an elongated body having at least one longitudinal ridge (1707).

5. The suture anchor of claim 4 wherein the plunger comprises a plurality of planar sides (1705) intersecting to form a plurality of longitudinal ridges.

6. The suture anchor of claim 1 wherein the elongated body comprises a radially enlarged head (1724).

7. The suture anchor of claim 6 wherein the elongated body comprises at least one flat surface (1720, 1722) interrupting the ridges.

8. The suture anchor of claim 7 wherein the at least one flat surface extends from the second transverse suture receiving passageway (1710, 1712) proximally to the head.

## Patentansprüche

1. Nahtanker, umfassend:
einen lang gestreckten Körper (1700), der ein proximales Ende (1702) und ein distales Ende (1704) aufweist,
wobei der Körper eine äußere Oberfläche aufweist, auf der sich radial erstreckende Knocheneingriffsrippen (1718, 1726) ausgebildet sind, wobei der Körper einen Längsdurchlass (1708), der sich von dem proximalen Ende hin zu dem distalen Ende erstreckt und eine Längsdurchlassachse (1706) und eine Innenwand definiert, und einen festen Kolben (1750), der ein proximales Ende und ein distales Ende aufweist, aufweist, **dadurch gekennzeichnet, dass** der Körper einen ersten quer verlaufenden Nahtaufnahmedurchlass (1714, 1716), der sich näher an dem proximalen Ende und den Längsdurchlass schneidend durch den Körper erstreckt, und einen zweiten quer verlaufenden Nahtaufnahmedurchlass (1710, 1712), der sich näher an dem distalen Ende und den Längsdurchlass schneidend durch den Körper erstreckt, aufweist, wobei der erste und zweite Nahtaufnahmedurchlass entlang der Längsdurchlassachse voneinander beabstandet sind; und der feste Kolben in einer axialen Translationsbeziehung zwischen einer ersten Position, in der das distale Ende des Kolbens proximal zu dem ersten quer verlaufenden Nahtaufnahmedurchlass ist, und einer zweiten Position, in der das distale Ende des Kolbens distal zu dem zweiten quer verlaufenden Nahtaufnahmedurchlass ist und das proximale Ende des Kolbens proximal zu dem ersten quer verlaufenden Nahtaufnahmedurchlass ist, innerhalb des Längsdurchlasses aufnehmbar ist, wobei der Kolben in der zweiten Position dazu bedienbar ist, Nahtabschnitte zusammenzudrücken, die in jeder der ersten und zweiten Queröffnung radial zwischen dem Kolben und der Innenwand des Längsdurchlasses aufgenommen sind.

2. Nahtanker nach Anspruch 1, der ferner einen dritten (1710, 1712) und einen vierten (1714, 1716) quer verlaufenden Nahtaufnahmedurchlass umfasst, die sich jeweils durch den Körper und den Längsdurchlass schneidend erstrecken, wobei der erste und dritte quer verlaufende Nahtaufnahmedurchlass ein distales Paar von Durchlässen ausbilden, die hin zu entgegengesetzten Seiten der Längsdurchlassachse gegeneinander versetzt sind , wobei der zweite und vierte quer verlaufende Nahtaufnahmedurchlass ein proximales Paar von Durchlässen ausbilden, die hin zu entgegengesetzten Seiten der Längsdurchlassachse gegeneinander versetzt sind.

3. Nahtanker nach Anspruch 2, wobei das distale Ende (1756) des Kolbens sich zu einer schmalen distalen Spitze verjüngt, die in der zweiten Position innerhalb des Längsdurchlasses enthalten ist.

4. Nahtanker nach Anspruch 1, wobei der Kolben einen lang gestreckten Körper umfasst, der mindestens eine Längsrippe (1707) aufweist.

5. Nahtanker nach Anspruch 4, wobei der Kolben mehrere plane Seiten (1705) umfasst, die sich schneiden, um mehrere Längsrippen auszubilden.

6. Nahtanker nach Anspruch 1, wobei der lang gestreckte Körper einen radial vergrößerten Kopf (1724) umfasst.

7. Nahtanker nach Anspruch 6, wobei der lang gestreckte Körper mindestens eine flache Oberfläche (1720, 1722) umfasst, welche die Rippen unterbricht.

8. Nahtanker nach Anspruch 7, wobei die mindestens eine flache Oberfläche sich ausgehend von dem zweiten quer verlaufenden Nahtaufnahmedurchlass (1710, 1712) proximal zu dem Kopf erstreckt.

## Revendications

1. Ancrage de suture comprenant :
un corps allongé (1700) présentant une extrémité proximale (1702) et une extrémité distale (1704), le corps présentant une surface extérieure avec des nervures d'engagement osseux (1718, 1726) formées sur celle-ci tout en s'étendant radialement, le corps comportant un passage longitudinal (1708) s'étendant de l'extrémité proximale vers l'extrémité distale et définissant un axe de passage longitudinal (1706) et une paroi intérieure, et un piston solide (1750) présentant une extrémité proximale et une extrémité distale,
**caractérisé en ce que** le corps comporte un premier passage transversal de réception de suture (1714, 1716) s'étendant à travers le corps à proximité de l'extrémité proximale et croisant le passage longitudinal, et un deuxième passage transversal de réception de suture (1710, 1712) s'étendant à travers le corps à proximité de l'extrémité distale et croisant le passage longitudinal, les premier et deuxième passages de réception de suture étant espacés le long de l'axe du passage longitudinal ;
le piston solide pouvant être reçu dans le passage longitudinal dans une relation de translation axiale entre une première position dans laquelle l'extrémité distale du piston est proche du premier passage transversal de réception de suture, et une deuxième position dans laquelle l'extrémité distale du piston est distale par rapport au deuxième passage transversal de réception de suture et l'extrémité proximale est proximale par rapport au premier passage transversal de réception de suture, le piston étant opérationnel dans la deuxième position pour comprimer des parties de suture reçues dans chacune des première et deuxième ouvertures transversales radialement entre le piston et la paroi intérieure du passage longitudinal.

2. Ancrage de suture selon la revendication 1, comprenant en outre des troisième (1710, 1712) et quatrième (1714, 1716) passages transversaux de réception de suture s'étendant respectivement à travers le corps et croisant le passage longitudinal, les premier et troisième passages transversaux de réception de suture formant une paire distale de passages décalés l'un par rapport à l'autre vers des côtés opposés de l'axe de passage longitudinal, les deuxième et quatrième passages transversaux de réception de suture formant une paire proximale de passages décalés l'un par rapport à l'autre vers des côtés opposés de l'axe de passage longitudinal.

3. Ancrage de suture selon la revendication 2, dans lequel l'extrémité distale (1756) du piston se rétrécit en une pointe distale étroite contenue dans le passage longitudinal dans la deuxième position.

4. Ancrage de suture selon la revendication 1, dans lequel le piston comprend un corps allongé présentant au moins une nervure longitudinale (1707).

5. Ancrage de suture selon la revendication 4, dans lequel le piston comprend une pluralité de côtés planaires (1705) se croisant pour former une pluralité de nervures longitudinales.

6. Ancrage de suture selon la revendication 1, dans lequel le corps allongé comprend une tête élargie radialement (1724).

7. Ancrage de suture selon la revendication 6, dans lequel le corps allongé comprend au moins une surface plane (1720, 1722) interrompant les nervures.

8. Ancrage de suture selon la revendication 7, dans lequel l'au moins une surface plane s'étend à partir du deuxième passage transversal de réception de suture (1710, 1712) de façon proximale jusqu'à la tête.
